(19) Europäisches Patentamt
European Patent Office
Office européen des brevets

(11) EP 2 921 855 A1

(12) EUROPEAN PATENT APPLICATION

(43) Date of publication:
**23.09.2015 Bulletin 2015/39**

(21) Application number: **14305396.5**

(22) Date of filing: **20.03.2014**

(51) Int Cl.:
***G01N 33/50*** *(2006.01)* ***A61P 35/00*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicants:
- **Centre National de la Recherche Scientifique (CNRS)**
  **75016 Paris (FR)**
- **UNIVERSITE PARIS DESCARTES**
  **75006 Paris (FR)**
- **INSTITUT NATIONAL DE LA SANTE ET DE LA RECHERCHE MEDICALE (INSERM)**
  **75013 Paris (FR)**

(72) Inventors:
- **Gavard, Julie**
  **75014 Paris (FR)**
- **Galan-Moya, Eva-Maria**
  **75014 Paris (FR)**

(74) Representative: **Regimbeau**
**20, rue de Chazelles**
**75847 Paris Cedex 17 (FR)**

(54) **Use of compounds inhibiting apelin / apj / gp130 signaling for treating cancer**

(57) Today, despite current advances in combinatorial therapies such as surgery, radiotherapy and chemotherapy, aggressive cancers remain fatal. Cancer stem-like cells (CSCs) may account for chemotherapy resistance and thus represent a promising therapeutic target. In this context, the present inventors identified essential intracellular pathways favoring the self-renewal and survival of CSCs. More precisely, the present inventors showed that the cytokine co-receptor GP130 acts as a co-receptor for Apelin / APJ signaling and that the interaction of Apelin with APJ/GP130 activates a dual signaling pathway involving the Akt/mTOR and STAT3 transcription factor, thereby promoting CSCs survival and self-renewal. They therefore propose to block these pathways in order to treat patients suffering from tumors containing CSCs, such as glioblastomas.



Printed by Jouve, 75001 PARIS (FR)

## Description

## BACKGROUND OF THE INVENTION

Increasing evidence suggests that initial cancer development is due to a rare population of cells, termed cancer stem cells (CSCs) (also known as "tumor-initiating cells", or "tumor side-populations", or "cancer stem-like cells") that are able to initiate and sustain this disease. These cells have indeed been demonstrated to be responsible for tumorigenesis, cancer metastasis, and cancer reoccurrence in particular cancers. Cancer stem cells have self-renewal capacity and they can differentiate into multiple cell types, although the equilibrium between self-renewal and differentiation potential shifts towards enhanced self-renewal, leading to limited differentiation capacity.

Conventional chemotherapies and radiotherapies are known to kill differentiated or differentiating cells, which form the bulk of the tumor (but that are unable to regenerate tumors). However, the population of cancer stem cells remains unaffected by said treatments and often causes a relapse of the disease.

In this context, it is imperative that anti-cancer therapies include strategies affecting preferentially CSCs. As a matter of fact, by targeting cancer stem cells, it will be possible to treat patients with aggressive and non-resectable tumors, as well as preventing tumor metastasis and recurrence.

Moreover, the identification of molecules that target CSCs in a selective manner, i.e., while sparing non-cancerous or normal stem cells, is critical to provide new anti-cancer drugs having few side effects.

It is therefore important to identify and validate pathways that are selectively implicated in cancer stem cell self-renewal and survival. Yet, though multiple pathways underlying properties of embryonic or adult stem cells have been already elucidated, few pathways have been identified for cancer stem cell self-renewal and survival.

Glioblastoma multiforme (GBM) is the most frequent and malignant primary tumor of the central nervous system in adults (60 000 cases/year in Europe and US). It has been proposed that GBM tumors derive from a small fraction of cells that constitute a reservoir of self-sustaining cells with the exclusive ability to self-renew and maintain the tumor, called glioblastoma stem-like cells (GSCs) (Singh *et al,* 2003). Regardless of the brain tumor ontogeny, GSCs might be involved in resistance to radiotherapies and contribute to tumor recurrence and aggressiveness (Bao *et al,* 2006). Furthermore, alterations in GSC levels affect tumor growth in experimental models of glioma (Piccirillo *et al,* 2006). Very few therapeutic protocols exist for this disease and, despite major clinical efforts, median survival rates from the time of diagnosis range between 12 and 15 months, and fewer than 3% of patients survive more than 5 years (Fisher *et al,* 2007).

In this context, the inventors identified pathways that regulate specifically the self-renewal and survival of CSCs - and in particular of GSCs. They now propose to screen for compounds that are able to block specifically these pathways, said compounds being efficient for treating patients with aggressive tumors, with minor side effects.

More precisely, the inventors highlight for the first time the essential interplay between the Akt/mTOR and STAT3 signaling pathways in self-renewal and survival of cancer stem-like cells such as GSCs. These pathways are activated by the endothelial-produced Apelin which operates through the APJ and GP130 membrane co-receptors. Thus, compounds impairing the protein interaction of Apelin on its coreceptors APJ/GP130 (thereby inhibiting the Akt/mTor and STAT3 pathways) in CSCs will lead to their apoptosis and thus refrain tumor progression and recurrence.

## LEGENDS OF THE FIGURES

**Figure 1. Analysis of the secretome content.**

**Figure 2. Endothelial production of Apelin regulates GSC integrity.**
**A.** RT-PCR for Apelin and Actin as a control in mRNA prepared from brain endothelial cells (EC) and patient-derived GSC#1. **B.** Apelin secretion was measured by ELISA in positive and negative templates (C+ and C-, respectively), and in HEK-293T (293T), EC and GSC#1 (TG1) conditioned media (CM). **C-D.** Quantification of neurosphere formation per field of view (NS/FOV) of GSC#1 grown in complete media (NS34 or C), in deprived medium (SF) or in SF supplemented with recombinant Apelin (Apelin) at 1 $\mu$M, unless specified. **E.** EC received non-silencing siRNA (sic) or siRNA targeting Apelin and Apelin mRNA levels were checked 3 days later by RT-PCR. **F.** Stable ECs expressing either a non-silencing shRNA (shC) or shRNA targeting Apelin were used to prepared conditioned media and secreted Apelin levels were estimated by ELISA. **G.** Quantification of neurosphere formation per field of view (NS/FOV) of GSC#1 grown in CM prepared from ECs that received either shRNA control or targeting Apelin.

**Figure 3. APJ profile of expression.**
**A.** mRNA levels were analyzed by RT-PCR in 16 GSCs for APJ, GP130, Sox2, Nestin, Oct4 and Actin. **B.** mRNA levels were analyzed by RT-PCR in GSC#10, either grown as neurosphere (NS) or induced in differentiation (Diff.)

for APJ, GP130, Sox2, Nestin, Oct4 and Actin. **C.** APJ expression was measured by indirect immunofluorescence and flow cytometry in GSC NS (green) or Diff (#, pink). Isotype control is shown in blue. **D-F.** mRNA levels were analyzed by RT-PCR for the indicated primers in GBM cell lines (U87, U138, U251 and LN229), mock or APJ-HA-transfected HEK-293T cells (293T), cancer stem-like cells isolated from GBM, Breast and Liver Cancer either grown as NS or Diff, human normal hematopoietic stem cells (HSC) and human fetal astrocytes (F Astro).

**Figure 4. Endothelial Apelin maintains GSC integrity through APJ signaling to mTOR.**
**A-B.** Western-blot analysis for phosphorylated Akt on Ser 473 (pAkt Ser), pS6, Total Akt, Total S6 and Tubulin in GSC#1 treated with complete media (NS34), deprived medium (SF), endothelial cell-conditioned media (EC-CM), or SF supplemented with Apelin (1 μM, APLN), for the indicated times. Alternatively, GSC#1 received EC-CM prepared from non-silencing shRNA (shC) or shRNA targeting Apelin. **C-D.** GSC#1 received non-silencing siRNA (siC) or siRNA targeting APJ. Knockdown efficiency was estimated by indirect immunofluorescence and flow cytometry (C) and GSC#1 were cultured with the resulting EC-CM for the indicated times, and analyzed by western-blots using the indicated antibodies (D). E. GSC#1 were pre-treated with APJ antagonist 45 min prior EC-CM or Apelin stimulation and protein lysates were analyzed by western-blots. **F.** Neurosphere assay was conducted in GSC#1 treated with siRNA as in (D) or with pharmacological APJ inhibitor as in (E), and number of neurosphere per field of view (NS/FOV) was quantified.

**Figure 5. Endothelial Apelin triggers GP130/STAT3 activation.**
**A.** Western-blot analysis for pY-STAT3, pS-STAT3 and Total STAT3 in GSC#1 treated with complete media (NS34), deprived medium (SF), endothelial cell-conditioned media (EC-CM), or SF supplemented with Apelin (1 μM), for the indicated times. **B.** GSC#1 received non-silencing siRNA (sic) or siRNA targeting APJ. GSC#1 were treated with EC-CM and analyzed by western-blots using the indicated antibodies (upper panel). Alternatively, GSC#1 received EC-CM prepared from non-silencing shRNA (shC) or shRNA targeting Apelin (lower panel). **C.** GSC#1 received non-silencing siRNA (siC) or siRNA targeting GP130, were stimulated with EC-CM three days later, and protein lysates were analyzed by western-blots, using the indicated antibodies. **D.** GSC#1 were pre-treated with GP130-blocking antibody 45 min prior EC-CM or Apelin stimulation and protein lysates were analyzed by western-blots. **E.** Neurosphere assay was conducted in GSC#1 treated with siRNA as in (C) or with GP130-blocking antibody as in (D).

**Figure 6. Apelin co-opts GP130 and APJ.**
**A.** STAT3 reporter activity was measured through luciferase-based assay in HEK-293T cells transfected with mock, APJ-, GP130-expression plasmid and both. Cells were not stimulated (nostim) or treated with Apelin (1 μM) for 6h, following vehicle (ctrl), APJ inhibitor or GP130-blocking antibody 45 min pre-treatment. **B.** BRET analysis was conducted in HEK-293T cells transfected with mock, APJ-, GP130-expression plasmid and both. Cells received Apelin (1 μM) for the indicated times, and data are presented as Delta mBRET on one representative experiment. **C-D.** Binding experiment as described in materials and methods in HEK-293T cells transfected with either APJ- or APJ- and GP130--expression plasmids (C) and in GSC#1 from membrane preparation (surface) or total lysates (D). **F.** Evolution of the tumor volume of glioblastoma xenograft mouse model that were obtained by subcutaneous injection of GSCsand were then treated with either PBS or an APJ antagonist (MM54).

**Figure 7. Scheme of the signaling pathways involved in CSCs properties.**

The present inventors identified that endothelial-produced Apelin operates through APJ and GP130 membrane receptors, and subsequently activates the Akt/mTOR and STAT3 intracellular signaling pathways. This molecular network is functional in cancer stem-like cells, and promotes self-renewal and survival, to ultimately maintain their integrity.

## DETAILED DESCRIPTION OF THE INVENTION

Apelin is a 13 amino-acid peptide known for its role in the cardiovascular and neuroendocrine systems. Apelin is reported to operate through a G-protein coupled receptor called "APJ", which is expressed by endothelial cells of various organs (such as heart, brain, kidney, lungs, etc.) and of a wide variety of tumor types (Rayalam S. et al, 2011). The function of the Apelin / APJ system and the downstream molecular mechanisms involved in cancer stem-like cells are not described yet. The present inventors showed that, surprisingly, the cytokine co-receptor GP130 acts as a co-receptor for Apelin / APJ signaling and that the interaction of Apelin with APJ/GP130 activates a dual signaling pathway involving the Akt/mTOR pathway and the STAT3 transcription factor, thereby promoting GSCs survival and self-renewal. Their results suggest that impairing the Apelin/APJ/GP130 axis inhibits GSCs survival and self-renewal, independently of the Apelin angiogenic activity on ECs.

The molecular axis involving Apelin/APJ/GP130 is therefore a suitable target to interfere with CSCs survival, and, in particular, with GSCs survival. The present invention proposes a methodology for the screening of Apelin/APJ/GP130 pathway inhibitors that target CSCs and are therefore efficient anti-cancer treatments. This invention is of high interest for future medical applications.

Importantly, the compound(s) identified by the screening method of the invention would specifically affect CSCs because only these cells are dependent upon the Apelin/APJ/GP130 pathway and the downstream activation of mTOR and STAT3.

In a first aspect, the present invention relates to a screening method for identifying compounds capable of inhibiting Cancer Stem Cells (CSCs) properties such as self-renewal and survival. This method comprises the steps of:

a) contacting cells expressing the co-receptors APJ and GP130 with the Apelin polypeptide and a candidate compound,

b) detecting the activation level of an Apelin-dependent signaling pathway in the cells obtained in step a),

c) comparing said activation level with the activation level of said cells contacted only with the Apelin polypeptide.

In a preferred embodiment, the said compound is selected if the activation level of an Apelin-dependent signaling pathway is impaired in the presence of said compound. In other words, the said compound is selected if the activation level of an Apelin-dependent signaling pathway is decreased in cells contacted with said compound and Apelin, as compared with the activation level of the same Apelin-dependent signaling pathway measured in the presence of Apelin alone.

***The Apelin polypeptide and its receptor, APJ.***

The Apelin gene (also known as APLN, APJ endogenous ligand, APEL and XNPEP2) encodes a pre-proprotein of 77 amino acids containing a signal peptide in the N-terminal region. After translocation into the endoplasmic reticulum and cleavage of the signal peptide, the proprotein of 55 amino acids generates several active fragments: a 36 amino acid peptide corresponding to the sequence 42-77 (Apelin 36), a 17 amino acid peptide corresponding to the sequence 61-77 (Apelin 17) and a 13 amino acid peptide corresponding to the sequence 65-77 (Apelin 13). In human, the proprotein Apelin has for example the SEQ ID NO:1 (NP_059109).

As used herein, the term "Apelin polypeptide" or "Apelin" is intended to refer to a polypeptide that comprises the C-terminal 13 amino acids of the Apelin proprotein (i. e., the polypeptide of SEQ ID NO: 2, corresponding to amino acids 65-77 of NP_059109) or a polypeptide that corresponds to the sequence 42-77 of the Apelin proprotein (Apelin 36) (i. e., the polypeptide of SEQ ID NO: 3). Preferably, it consists of SEQ ID NO:2 (corresponding to amino acids 65-77 of NP_059109).

The Apelin polypeptide used in the method of the present invention can be obtained by any conventional means. For example, it can be obtained by suppliers of biochemical molecules (Santa Cruz, Cayman, Sigma Aldrich, etc.). It can also be synthesized chemically by standard peptide synthesis techniques. For the purposes of this invention, when chemically synthesized, the apelin-13 peptide may comprise a pyroglutamate rather than a glutamic acid residue in the N-terminal position. As an alternative to standard peptide synthesis, the Apelin polypeptide can be produced using standard recombinant DNA techniques. For example, a nucleic acid molecule encoding the polypeptide can be cloned into an expression vector, the expression vector can be introduced into a host cell, and the Apelin polypeptide can be expressed in the host cell. The Apelin polypeptide can then be isolated from the cells by an appropriate purification scheme using standard polypeptide purification techniques.

The Apelin polypeptide has been involved in several physiological functions depending on the cellular localization of its receptor, APJ. These include cardiovascular regulation, fluid homeostasis, modulation of the adipoinsular axis, and HIV coreceptor function in *vitro* (Pitkin et al, Pharmacological Review 2010). Apelin has been implicated in cancer as a pro-angiogenic factor, although its exact role is still matter of debate (Rayalam S. et al, 2011 and Kidoya H., 2012). Apelin/APJ signaling is known to activate the mTor pathway (Masri B. et al, 2004).

APJ (also known as Apelin receptor, AR, Angiotensin receptor-like 1, G-protein coupled receptor APJ, APLNR, AGTRL, APJ, APJR, and HG11) is the cognate receptor for Apelin (Lee DK. et al, J. Neurochem. 2000). It belongs to the G protein-coupled receptor family. APJ is related to the angiotensin II receptor and has been described as a co-receptor involved in the mediation of HIV-1 neuropathogenesis, cardiovascular and neuroendocrine functions (Pitkin et al, Pharmacological Review, 2010). In human, APJ has for example the SEQ ID NO:4 (NP_005152) and is encoded by SEQ ID NO:5 (NM_005161).

### *The GP130 co-receptor*

The GP130 protein (also known as Glycoprotein 130, IL6ST, IL6-beta, Interleukin-6 receptor subunit beta, IL-6 receptor subunit beta, IL-6R subunit beta, IL-6R-beta, IL-6RB, IL6RB, CDw130, Interleukin-6 signal transducer, Membrane glycoprotein 130, Oncostatin-M receptor subunit alpha or CD130) is a trans- membrane protein cytokine receptor. More specifically, GP130 is known to facilitate cytokine-driven signaling, such as the ones induced by IL-6, EPO and LIF. It has no intrinsic tyrosine kinase activity. Instead, it is phosphorylated on tyrosine residues after complexing with other proteins. This phosphorylation leads to association with JAK/Tyk tyrosine kinases and STAT protein transcription factors, such as STAT-3. Structurally, GP130 is composed of five fibronectin type-III domains and one immunoglobulin-like C2-type domain in its extracellular portion. In human, it has the sequence SEQ ID NO:6 (Gene Bank number: P40189.2) and is encoded by SEQ ID NO:7 (NM_000565.3).

GP130 is known to activate the STAT3 pathway. This pathway is implicated in cell proliferation, migration and survival, and is further reported activated in cancer stem-like cells (Chautard E. et al, 2010), and recently in GSCs (Kim E. et al, 2013). However, the present inventors describe for the first time the functional interplay between APJ/GP130 and Apelin.

### *Cells expressing the APJ and GP130 receptors*

The screening method of the invention may use any cells expressing the co-receptors APJ and GP130. Expression of these co-receptors can be endogenous or exogenous. In a preferred embodiment, the screening method of the invention uses cells expressing high levels of the said receptors. Although it is not excluded to use primary cells, cultured cells from conventional cell lines are however preferred, as their behavior is fully characterized, and they can be easily transfected in order to express exogenous polypeptides.

In a more preferred embodiment, the method of the invention uses recombinant cells issued from conventional cell lines that have been transfected with exogenous nucleotide vectors expressing the co-receptors APJ and GP130. In an even more preferred embodiment, these cells do not endogenously express the co-receptors APJ and GP130, so as to ensure that the Apelin-dependent activation in GSCs requires the presence of the two co-receptors APJ and GP130 and not of only one of them.

These conventional cell lines are for example HEK293T cells, HeLa cells, COS-7 cells, CHO cells, U87-MG, and LN229 cells.

A particular cell line of interest is for example the APJ-expressing cell line of Millipore which is made in the Chem-5 host and supports high levels of recombinant APJ expression on the cell surface. This cell line advantageously contains optimized levels of a recombinant promiscuous G protein to couple the receptor to the calcium signaling pathway.

Methods to generate recombinant cells expressing APJ and GP130 are well-known in the art. For examples, conventional molecular biology, microbiology and recombinant DNA techniques can be used [see Sambrook, Fitsch & Maniatis, Molecular Cloning: A Laboratory Manual, Second Edition (1989) Cold Spring Harbor Laboratory Press, Cold Spring Harbor, N.Y.), Current Protocols in Molecular Biology, John Wiley & Sons, Inc. (1994)].

In particular, nucleotide vectors expressing the APJ and GP130 co-receptors may be transfected in the cells by means of any "transfection" system (for example by lipofection, by infection, by calcium phosphate-DNA precipitation or by electroporation), and the translation of the polynucleotide encoding said co-receptors is induced.

The term "vector", as used herein, is intended to refer to a nucleic acid molecule capable of carrying another nucleic acid. One type of vector is a "plasmid", which refers to a circular double stranded DNA loop into which additional DNA segments may be ligated. Another type of vector is a viral vector, wherein additional DNA segments may be ligated into the viral genome. Certain vectors are capable of autonomous replication in a host cell into which they are introduced (e. g., bacterial vectors having a bacterial origin of replication and episomal mammalian vectors). Other vectors (e. g., non-episomal mammalian vectors) can be integrated into the genome of a host cell upon introduction into the host cell, and thereby are replicated along with the host genome.

Certain vectors are capable of directing the expression of genes to which they are operatively linked. Such vectors are referred to herein as "recombinant expression vectors" (or simply, "expression vectors"). In general, expression vectors of utility in recombinant DNA techniques are in the form of plasmids. In the present specification, "plasmid" and "vector" may be used interchangeably as the plasmid is the most commonly used form of vector. However, the invention is intended to include such forms of expression vectors, such as bacterial plasmids, YACs, cosmids, retrovirus, EBV-derived episomes, and all the other vectors that the skilled man will know to be convenient for ensuring the expression of the co-receptors GP130 and APJ.

Transformation of these vectors into the cells can be carried out by any known method for introducing polynucleotides into a host cell. Such methods are well known of the man skilled in the art and include e.g., dextran-mediated transformation, calcium phosphate precipitation, polybrene-mediated transfection, protoplast fusion, electroporation, encapsulation of the polynucleotide into liposomes, biolistic injection and direct microinjection of DNA into nuclei.

In a particular embodiment, the cells used in the method of the invention are HEK-293T stably expressing the APJ and the GP130 co-receptors of SEQ ID NO:4 and 6 respectively. HEK-293T can be transfected for example with plasmids encoding these polypeptides using the Turbofect system, using the manufacturer recommendations (for detailed explanations, see examples below).

The method of the invention comprises a first step of "contacting cells expressing the co-receptors APJ and GP130 with the Apelin polypeptide and a candidate compound".

The Apelin polypeptide may be added after, simultaneously, or before the candidate compound.

In a preferred embodiment, the recombinant cells expressing the co-receptors APJ and GP130 are contacted with the Apelin polypeptide after being contacted with the candidate compound. The tested compound, if selected, will be for example able to impair the binding of Apelin on its binding site on the APJ/GP130 co-receptors or to down regulate the expression of APJ or GP130.

In another preferred embodiment, the recombinant cells expressing the co-receptors APJ and GP130 are contacted with the Apelin polypeptide after being contacted with the candidate compound. The tested compound, if selected, will be for example able to mask the binding site of Apelin on APJ/GP130 without activating these co-receptors (therefore acting as an antagonist of the Apelin).

If Apelin and the candidate compound are put in contact simultaneously with the cells expressing APJ/GP130, both phenomenon are likely to occur.

***Cancer Stem Cells (CSCs) and CSCs properties***

As used herein, the term "Cancer stem cells" or "CSCs" refers to cancer cells that possess the properties of normal stem cells, specifically the ability to differentiate into multiple cell types (potency), and the ability to go through numerous cycles of cell division while maintaining in an undifferentiated state (self-renewal).

These properties may be tested by various methods such as clonogenic or sphere assays, in which single cells are assessed for their ability to differentiate and self-renew (Thomson SP, Cancer Research, 1982). Also, clonal cell transplantation systems can be used (Nakauchi et al, Ann N Y Acad Sci. 2001), as well as their ability to initiate tumor formation in *vivo.*

CSCs can be identified by various means. For example, it is possible to identify them by using distinctive set of cell surface markers including CD133 (also known as PROM1), CD44, CD24, EpCAM (epithelial cell adhesion molecule, also known as epithelial specific antigen, ESA), THY1 and ATP-binding cassette B5 (ABCB5) (Al-Hajj et al, PNAS 2003). Alternatively, it is possible to identify CSCs by letting them grow in the absence of serum and without attachment to culture plates (differentiated cells fail to survive under the same conditions). Also, CSCs can be identified by their characteristic slow-cycling and quiescent properties (Horan PK, Methods Cell Biol. 1990).

In a preferred embodiment, the Cancer Stem Cells targeted by the compound of the invention are those present in breast cancer, head and neck cancer, lung cancer, ovarian cancer, pancreatic cancer, colorectal carcinoma, cervical cancer, sarcomas, brain tumors, renal cancer, melanoma and skin cancer, prostate cancer, gastric cancer, multiple myeloma, leukemia and lymphoma.

In a particularly preferred embodiment, Cancer Stem Cells targeted by the compound of the invention are Gliobastoma Stem Cells (GSCs). Gliobastoma Stem Cells are CSCs present in brain tumors. Biomarkers of GSCs are for example CD133, podoplanin, CD15, A2B5, filament markers (nestin), RNA-binding proteins (Musashi-1) and transcription factors (BMI1, SOX2, Id1, and Oct-4) (for a review, see Dahlrot RH. et al, Int. J. Clin. Exp. Pathol. 2013).

***Compound to be tested***

The compound to be tested in the method of the invention is of any kind. In a preferred embodiment, it is selected from the group consisting of: an anti-sense nucleic acid, a receptor decoy, an aptamer, an antibody and a small molecule antagonist.

As used herein, the term "anti-sense nucleic acid" refers to a polynucleotide that is specific for a sequence encoding the Apelin, GP130 or APJ polypeptides, or a portion of one of those sequences. The nucleic acid sequences encoding these proteins are described herein (SEQ ID NO:5 and 7). These sequences are furthermore available on public databases, such as the GenBank database operated by the NCBI.

A person skilled in the art would be able to design, make and use suitable anti-sense molecules, based on these sequences, without undue experimentation. The anti-sense nucleic acid may be, e.g., an oligonucleotide, or a nucleic acid comprising an anti-sense sequence that is operably linked to an expression control sequence. The use of anti-sense nucleic acids to down-regulate the expression of a particular protein in a cell is well known in the art. An anti-sense nucleic acid molecule may comprise a nucleotide sequence that is complementary to the coding strand of another nucleic acid molecule (e.g., an mRNA sequence), or to a portion thereof, and accordingly is capable of hydrogen bonding to the coding strand of the other nucleic acid molecule. Alternatively, anti-sense sequences can be complementary to a sequence found in the 5' or 3' untranslated region of the mRNA or a region bridging the coding region and an untranslated region (e.g., at the junction of the 5' untranslated region and the coding region). The anti-sense nucleic acid can be complementary in sequence to a regulatory region of the gene encoding the mRNA, for instance a transcription initiation sequence or regulatory element, or a splice site. In one embodiment, an anti-sense nucleic acid is designed so as to be complementary to a region preceding or spanning the initiation codon on the coding strand or in the 3' untranslated region of an mRNA.

The anti-sense nucleic acid of the invention is preferably an RNA, such as a short interfering RNA (siRNA), a double-stranded RNA (dsRNA), a micro-RNA (miRNA), a short-hairpin RNA (shRNA).

As used herein, the term "receptor decoy" refers to a molecule that will bind to an Apelin polypeptide and prevent the apelin polypeptide from participating in its native signaling pathway (s). A receptor decoy is intended to encompass a soluble receptor for apelin (or fragment thereof) which is capable of binding apelin and inhibiting apelin from signaling through its native signaling pathway (s).

As used herein, the term "aptamer" relates to oligonucleic acid or peptide molecules that bind specifically to a specific target molecule. It preferably refers to an oligonucleic acid. Aptamers can be selected *in vitro* by the SELEX process from very large populations of random sequence oligomers (Ellington et Szostak, Nature 1990). This well-established methodology selects aptamers based on their affinity for a specific target molecule.

In a more preferred embodiment, this tested compound is an antibody or a functional antibody fragment impairing the interaction of Apelin with GP130 and APJ. Said antibody or functional fragment thereof can preferably bind Apelin, GP130 or APJ.

The term "antibody" as used herein designates a polypeptide that exhibit binding specificity to a specific antigen. More particularly, an antibody (or "immunoglobulin") consists of a glycoprotein comprising at least two heavy (H) chains and two light (L) chains inter-connected by disulfide bonds. Each heavy chain comprises a heavy chain variable region (or domain) (abbreviated herein as $V_H$) and a heavy chain constant region (hereafter $C_H$). Heavy chains are classified as gamma, mu, alpha, delta, or epsilon, and define the antibody's isotype as IgG, IgM, IgA, IgD, and IgE, respectively. The $C_H$ region of the immunoglobulin IgG, IgD, and IgA (y, $\delta$ and $\alpha$ chains respectively) comprises three domains (CH1, CH2, and CH3) and a hinge region for added flexibility, while the $C_H$ region of the immunoglobulin IgM and IgE contains 4 domains (CH1, CH2, CH3, and CH4). An "epitope" is the site on the antigen to which an antibody binds. It can be formed by contiguous residues or by non-contiguous residues brought into close proximity by the folding of an antigenic protein.

As used herein, the term "antibody fragments" intends to designate Fab, Fab', $F(ab')_2$, scFv, dsFv, ds-scFv, dimers, minibodies, diabodies, and multimers thereof and bispecific antibody fragments. Antibodies can be fragmented using conventional techniques. For example, $F(ab')_2$ fragments can be generated by treating the antibody with pepsin. The resulting $F(ab')_2$ fragment can be treated to reduce disulfide bridges to produce Fab' fragments. Papain digestion can lead to the formation of Fab fragments. Fab, Fab' and $F(ab')_2$, scFv, dsFv, ds-scFv, dimers, minibodies, diabodies, bispecific antibody fragments and other fragments can also be synthesized by recombinant techniques.

A "functional fragment" of an antibody means in particular an antibody fragment as defined above, with the same binding activity as the parental antibody.

In the context of the present invention, an antibody is said to "recognize" or "bind" a peptide having a define sequence if said antibody has an affinity constant $K_a$ (which is the inverted dissociation constant, i.e. $1/K_d$) higher than $10^6$ $M^{-1}$, preferably higher than $10^7$ $M^{-1}$, more preferably higher than $10^8$ $M^{-1}$ for said peptide. Also, in the context of the present invention, an antibody is said to "specifically bind" or to "specifically recognize" a peptide if said antibody has an affinity constant $K_a$ higher than $10^7$ $M^{-1}$, preferably higher than $10^8$ $M^{-1}$, more preferably higher than $10^9$ $M^{-1}$ for said peptide and even more preferably higher than $10^{10}$ $M^{-1}$ for said peptide and has an affinity constant $K_a$ lower than $10^5$ $M^{-1}$ for all the other peptide.

The affinity constant which is used to characterize the binding of antibodies (Ab) to a peptide or an antigen (Ag) is the inverted dissociation constant defined as follows:

$$\mathrm{Ab} + \mathrm{Ag} \rightleftharpoons \mathrm{AbAg}$$

$$K_a = \frac{[\mathrm{AbAg}]}{[\mathrm{Ab}]\,[\mathrm{Ag}]} = \frac{1}{K_d}$$

This affinity can be measured for example by equilibrium dialysis or by fluorescence quenching, both technologies being routinely used in the art.

The tested compound may be a monoclonal or a polyclonal antibody. This antibody may be of any species of origin, including (for example) mouse, rat, rabbit, horse, or human, or may be chimeric antibodies.

A "polyclonal antibody" as used herein, refers to an antibody that is obtained from different B cells. It may be produced by standard antibody production methods, for example by i) immunizing a suitable animal (e.g., rabbit, goat, etc.) with the phosphorylated protein of the invention or with an immunogenic peptide , ii) collecting immune serum from the animal, and iii) separating the polyclonal antibodies from the immune serum, in accordance with known procedures.

A "monoclonal antibody", as used herein, means an antibody arising from a nearly homogeneous antibody population. In other words, a monoclonal antibody consists of a homogeneous antibody arising from the growth of a single cell clone (for example a hybridoma, a eukaryotic host cell transfected with a DNA molecule coding for the homogeneous antibody, a prokaryotic host cell transfected with a DNA molecule coding for the homogeneous antibody, etc.) and is characterized by heavy chains of one and only one isotype and subtype, and light chains of only one type. The monoclonal antibodies of the invention may be produced in a hybridoma cell line according to the well-known technique of Kohler and Milstein, Nature 1975; Kohler and Milstein, Eur. J. Immunol. (1976); see also, CURRENT PROTOCOLS IN MOLECULAR BIOLOGY, Ausubel et al. (1989).

In a preferred embodiment, the compound of the invention is able to block the interaction of Apelin on its co-receptors APJ and GP130. In another preferred embodiment, said compound is able to block the Apelin-induced interaction of GP130 with APJ.

By "blocking an interaction", it is herein preferably meant that the said interaction cannot physically occur in the presence of the compound of the invention.

In another preferred embodiment, the compound of the invention is able to block both the mTOR and the STAT3 signaling pathways.

By "blocking a signaling pathway", it is herein meant that the said signaling pathway cannot be activated in the presence of the compound of the invention.

By blocking these interactions and/or signaling pathways, the compound of the invention will be able to inhibit CSC properties (e.g., CSCs potency, self-renewal and survival), and, consequently, will impair CSC proliferation and cancer progression.

In a preferred embodiment, the tested compound is a small molecule that is known to inhibit the interaction between APJ and Apelin. This small molecule is for example MM54 (Macaluso NJ et al., 2011, ChemMedChem, 6 (6): 1017-23), which blocks Apelin-induced signaling and self-renewal in GSCs.

In another preferred embodiment, the tested compound is an anti-sense nucleic acid such as a siRNA or a shRNA, said compounds having the following sequences:

siRNA specific for APJ: CCAUCAUGCUGACCUGUUACUUCUU, GACAACCAGUCUGAGUGUGAGUACA (SEQ ID NO:10),
ACUAUGACUGGCCCUUUGGGACCUU (SEQ ID NO:11)
siRNA specific for Apelin: GGUGCAGCCCAGAGGGUCAAGGAAU (SEQ ID NO:12),
CCUCUCCCAUAAGGGACCCAUGCCU (SEQ ID NO:13),
CCUGAUGCCGCUUCCCGAUGGGAAU (SEQ ID NO:14)
shRNA specific for Apelin: TAACATTCTGTGATTCTTGGC (SEQ ID NO:15),
TTACAAACATTGAACACAGGG (SEQ ID NO:16),
AACAGGGCCTTAATATCTTTG (SEQ ID NO:17),
AAGCAGACCAATCTATGGAGG (SEQ ID NO:18),
TTTCTCTGCATTCTTCCCTGG (SEQ ID NO:19)
siRNA specific for GP130: CUCACUUGCAACAUUCUUA (SEQ ID NO:20)

In another preferred embodiment, the tested compound is an antibody that is known to inhibit the interaction between APJ and Apelin.

***Apelin-dependent*** *signaling pathway*

The method of the invention comprises a step of detecting the activation level of at least one "Apelin-dependent signaling pathway" (step b).

By "Apelin-dependent signaling pathway", it is herein meant a signaling pathway whose activation is induced by Apelin. In the context of the invention, this signaling pathway is preferably activated by the interaction of Apelin with the membrane receptors APJ and GP130.

In a preferred embodiment, the detecting step b) comprises the detection and/or measurement of the activation level of the APJ receptor. This activation level can be assessed by any conventional means.

For example, activation level of the APJ transmembrane receptor can be assessed by measuring Calcium influx in cells expressing APJ coupled to a protein G receptor (Chemiscreen® from Millipore, Fluo4NW from Life Technologies), or by measuring the content of phosphorylated extracellular signal-regulated kinase (pERK) or Akt (pAkt) (Scimia MC, Nature letters 2012).

Alternatively, activation of the APJ receptor can be assessed by studying the recruitment of the scaffold molecule beta-arrestin2 to APJ. Said recruitment can be detected by any conventional means. Preferably, it is measured by immunofluorescence and microscopy analysis, co-immunoprecipitation, Fluorescence Resonance Energy Transfert (FRET) or by Bioluminescence Resonance Energy Transfert (BRET). These technologies are well known in the art.

Beta-arrestin2 is a scaffold protein, recruited upon GPCR activation and is involved in modulating GPCR trafficking and signaling (Shenoy SK et al., 2011). Beta-arrestin 2 is recruited to the plasma membrane upon activation of the membrane receptor APJ. APJ binding induces a global conformational change that involves the movement of the two arrestin domains and the release of its C-terminal tail that contains clathrin and AP2 binding sites. This conformational change can be reflected by Bioluminescence Resonance Energy Transfert (BRET) by using a vector expressing i) the bioluminescent *Renilla* luciferase (RLUC), ii) the beta-arrestin2 protein of SEQ ID NO:8 (P32121.2) and iii) the yellow-emitting GFP mutant YFP. As a matter of fact, the emission spectrum of RLUC is sufficiently broad to provide good excitation of YFP.

In a specific embodiment of the method of the invention, the cells co-expressing APJ and GP130 are transfected by the RLuc-βarrestin2-YFP and subsequently stimulated with Apelin in the presence or in the absence of the tested compound. Light emission is detected (460-500 nm for RLUC and 510-550 nm for YFP) using conventional means. The BRET signal measuring beta-arrestin conformational change due to its recruitment to APJ is then determined as the ratio of the light emitted by YFP to that by RLUC. These values are preferably corrected by subtracting the background BRET RLUC signals detected when RLUC-beta-arrestin-YFP is expressed alone.

In this specific embodiment, Apelin-dependent activation of the APJ receptor is detected when the BRET signal is significantly increased in the presence of Apelin, as compared to control samples without Apelin polypeptide. Thus, the tested compound will be selected if, in the comparison step c), the BRET signal observed in the presence of Apelin is significantly decreased when said compound is added.

In one embodiment, the activation of the APJ receptor is assessed by assessing the activation status of an APJ-dependent signaling pathway. This APJ-dependent signaling pathway is for example the Akt/mTOR and ERK signaling pathways, calcium influx, and STAT3 activation.

In the context of the present invention, "the APJ-dependent signaling pathway" encompasses the APJ receptor itself, and/or a signaling pathway which is induced downstream of this receptor (e.g., the Akt/mTOR signaling pathway).

The mammalian target of rapamycin (mTOR) is an atypical serine/threonine kinase that is present in two distinct complexes, mTOR complex 1 and mTOR complex 2. The mTOR complex 1 (mTORC1) is composed of mTOR, Raptor, GβL (mLST8), and Deptor and is partially inhibited by rapamycin. mTORC1 integrates multiple signals reflecting the availability of growth factors, nutrients, or energy to promote either cellular growth when conditions are favorable or catabolic processes during stress or when conditions are unfavorable. Growth factors and hormones (e.g. insulin) signal to mTORC1 via Akt, which inactivates TSC2 to prevent inhibition of mTORC1. Alternatively, low ATP levels lead to the AMPK-dependent activation of TSC2 and phosphorylation of raptor to reduce mTORC1 signaling. Amino acid availability is signaled to mTORC1 via a pathway involving the Rag and Ragulator (LAMTOR1-3) proteins. Active mTORC1 has a number of downstream biological effects including translation of mRNA via the phosphorylation of downstream targets (4E-BP1 and p70 S6 Kinase), suppression of autophagy (Atg13, ULK1), ribosome biogenesis, and activation of transcription leading to mitochondrial metabolism or adipogenesis. On another hand, the mTOR complex 2 (mTORC2) is composed of mTOR, Rictor, GβL, Sin1, PRR5/Protor-1, and Deptor and promotes cellular survival by activating Akt. mTORC2 also regulates cytoskeletal dynamics by activating PKCdelta and regulates ion transport and growth via SGK1 phosphorylation.

Akt, which exists as multiple isoforms, is one of the principle kinases activated by phosphoinositide 3-kinase (PI3K). Activation of PI3K results in the generation of phosphatidylinositol (3,4,5)-triphosphate (PIP3). These lipid second messengers bind to the pleckstrin homology domain of Akt to promote its translocation to the plasma membrane for activation via phosphorylation at Thr308 and Ser473 by PDK1 and the mTORC2 complex, respectively. Phosphorylation at both these sites is required for full activation of Akt Ser/Thr kinase activity. Akt phosphorylates over 50 known substrates, including GSK3b, p70S6K, 4EBP1, AS160, PRAS40, TSC 1, TCS 2, Raf-1, Bad, the FOXO family of transcription factors, and PFK2.

In the context of the invention, the Akt/mTOR signaling pathway is said to be activated for example when known substrates of either Akt or mTOR kinases (such as GSK3b, p70S6K, 4EBP1, AS160, PRAS40, TSC 1, TCS 2, Raf-1, Bad, the FOXO family of transcription factors, and PFK2) are substantially phosphorylated, as compared to control samples provided by kit manufacturers. Alternatively, the Akt/mTOR pathway is said to be activated when for example, Akt is phosphorylated on both Thr308 and Ser473, when mTOR is phosphorylated, or when S6, a substrate for p70S6K, is phosphorylated.

Akt activation is preferably assessed by conventional assays such as ELISA or Western Blots. For this purpose, commercial kits are available, for example the Akt pathway activation Profile InstantOne™ from Affymetrix eBioscience, the Alphascreen® assay from PerkinElmer. Anti-phosphorylated Akt antibodies are also available (Cell Signaling technology).

mTor activation is preferably assessed by ELISA or Western Blots. It can moreover be measured by cell-based imaging using antibodies anti-phospho mTOR (Pierce) or flow cytometry for pAkt or pS6 (Cell Signaling Technology).

In another embodiment, the detecting step b) of the method of the invention comprises the detection and/or measurement of the activation level of the GP130 receptor. Activation level of GP130 can be directly measured by conventional means, for example by ELISA or western blot using antibodies recognizing specifically the Ser 782-phosphorylated form of GP130 (Santa Cruz).

In a preferred embodiment, GP130 activation level is assessed by measuring the activation level of a GP130-dependent signaling pathway. This GP130-dependent signaling pathway is for example the JAK/STAT3 signaling pathway. Additionally, MAPK and PI3K could be activated.

In the context of the present invention, "the GP130-dependent signaling pathway" encompasses the GP130 receptor itself, and/or a signaling pathway which is induced downstream of this receptor (e.g., the STAT3 signaling pathway).

The activation level of the STAT3 signaling pathway can be measured by any conventional means. Commercial kits, such as Flowcellect™ (Millipore), are available in this purpose. Also, the skilled person may detect STAT3 activation level by western blot or by ELISA using an antibody which is specific to Tyr705- and Ser727-phosphorylated STAT3 (supplied for example by Cell Signaling Technology).

In a specific embodiment, the activation level of the STAT3 signaling pathway is detected by measuring the expression level of a reporter protein (e.g., a luminescent or a fluorescent reporter protein), whose expression is operatively linked to the STAT3 promoter of SEQ ID NO:9. The skilled person is well aware of the specific conditions that are to be used for this assay. Particular conditions are nevertheless precisely disclosed in the experimental part below. Briefly, plasmids expressing the firefly luciferase constructs downstream of STAT3 promoter can be transfected into cells co-expressing GP130 and APJ, and these cells can be treated with Apelin (preferably 24 hours after transfection). The STAT3-dependent luciferase signal can be measured once the candidate compound is added. The firefly luciferase signal should be observed only in the presence of a functional interplay between Apelin, APJ and GP130.

In this specific embodiment, activation of the GP130 receptor is detected in the presence of Apelin when the activation level of the STAT3 pathway, and, in particular, the expression level of the reporter protein placed under control of the STAT3 promoter, is significantly increased as compared to control samples without Apelin polypeptide. Thus, the tested compound will be selected if, in the comparison step c), the expression of the reporter protein under control of the STAT3 promoter observed in the presence of Apelin is significantly decreased when said compound is added.

In another embodiment, the detecting step b) of the method of the invention comprises the detection and/or measurement of the physical interaction between the co-receptors APJ and GP130. Detection can be achieved through imagery- and/or biochemical-based approaches.

In a preferred embodiment, the detecting step b) of the method of the invention comprises i) the detection and/or measurement of a GP130-dependent signaling pathway (e.g., of the GP130 receptor or of the STAT3 signaling pathway), and ii) the detection and/or measurement of the activation level of an APJ-dependent signaling pathway (e.g., of the APJ receptor or of the Akt/mTOR signaling pathway). These activation levels can be assessed by any conventional methods, as mentioned previously.

In this preferred embodiment, the tested compound will be selected if, in the comparison step c), i) the activation level, in presence of Apelin, of the GP130 receptor or of the GP130-dependent signaling pathway and ii) the activation level, in presence of Apelin, of the APJ receptor or of the APJ-dependent signaling pathway is significantly decreased when said compound is added.

In this specific embodiment, step b) of the method of the invention may contain any combination of these assays, provided that both APJ- and GP130-dependent signaling pathways are assessed. In particular, it is possible to measure, in cells co-expressing APJ and GP130, in the presence of the Apelin polypeptide and optionally the tested compound:

either i) the activation level of the GP130 receptor and the activation level of the APJ receptor,

ii) the activation level of the GP130 receptor and the activation level of an APJ-dependent signaling pathway (e.g., of the Akt/mTOR signaling pathway), or

iii) the activation level of a GP130-dependent signaling pathway (e.g., of the STAT3 signaling pathway) and the activation level of the APJ receptor, or

iv) the activation level of a GP130-dependent signaling pathway (e.g., of the STAT3 signaling pathway) and the activation level of an APJ-dependent signaling pathway (e.g., of the Akt/mTOR signaling pathway).

In the method of the invention, the assessment of the activation status of the two signaling pathways may be performed in any order, or simultaneously. In other words, assessment of the activation level of GP130 or of the GP130-dependent signaling pathway can be performed simultaneously, before or after the activation level of APJ or of the APJ-dependent signaling pathway is assessed.

Yet, in a preferred embodiment, assessment of the activation level of GP130 or of the GP130-dependent signaling pathway is performed before the activation level of APJ or of the APJ-dependent signaling pathway is assessed.

In another preferred embodiment, the measurement of the activation level of APJ or of the APJ-dependent signaling pathway is performed only if the tested compound significantly decreases the Apelin-dependent activation of GP130 or of the GP130-dependent signaling pathway.

In practice, the activation status of the two signaling pathways may be performed independently. Consequently, the skilled person may use different cells for assessing the two different signaling pathways, provided that they express GP130 and APJ. Alternatively, the activation of the two signaling pathways may be assessed on the same cells.

In a preferred embodiment, the detecting step b) of the method of the invention comprises the detection and/or measurement, in cells co-expressing APJ and GP130, in the presence of the Apelin polypeptide and optionally the tested compound, of:

b1) the activation level of the STAT3 signaling pathway, and

b2) the beta-arrestin2 recruitment to APJ.

More precisely, the detecting step b) of the method of the invention comprises the steps of:

b1) measuring, in cells co-expressing APJ and GP130, in the presence of the Apelin polypeptide and optionally the tested compound, the expression of a reporter protein whose expression is operatively linked to the STAT3 promoter of SEQ ID NO:9,
and

b2) measuring, in cells co-expressing APJ and GP130, in the presence of the Apelin polypeptide and optionally the tested compound, the Apelin-dependent beta-arrestin2 recruitment to APJ, preferably by BRET.

In a preferred embodiment, the two steps b1) and b2) are performed in this order (b2 following b1). More precisely, the step b2) is performed only if the tested compound significantly decreases the Apelin-dependent activation level of the reporter protein expression measured in step b1). As a matter of fact, the STAT3 pathway integrates all the tested signaling pathways. Hence, if a compound does not block the Apelin-dependent STAT3 activation, then this compound is unlikely to affect GSCs properties, although it may be efficient to affect the APJ-dependent ERK activation.

In the context of the invention, it is meant that the activation level of the receptors or of the signaling pathway depending thereof is "significantly increased" in the presence of Apelin (and in the absence of the tested compound) if the value measured in step b), b1) or b2) in the recombinant cells expressing GP130 and APJ in presence of Apelin is 2 fold superior, preferably 4 fold, and more preferably 6 fold superior to the value measured in these cells in absence of Apelin (and in absence of the tested compound). Conversely, it is meant that the activation level of the receptors or of the signaling pathway depending thereof is "significantly decreased" in the presence of the tested compound if the value measured in step b), b1) or b2) in the recombinant cells expressing GP130 and APJ in presence of Apelin is 2 fold inferior, preferably 4 fold, and more preferably 6 fold inferior to the value obtained in these cells in absence of the tested compound (but in presence of Apelin).

### *Therapeutic use of the selected compounds*

In another aspect, the present invention relates to a method to inhibit CSC properties (e.g., CSCs potency, self-renewal and survival), said method comprising the step of contacting these cells with a compound that is able to:

a) inhibit the physical interaction of Apelin on its coreceptors APJ and GP130, or

b) inhibit the physical interaction of the coreceptors APJ and GP130, or

c) inhibit simultaneously the mTOR and STAT3 signaling pathways.

This method can be performed *in vitro* or *in vivo.*

This compound is preferably an anti-sense nucleic acid, a small chemical molecule or an antibody.

In a preferred embodiment, this compound is the small molecule MM54 (Macaluso NJ et al., 2011, ChemMedChem, 6 (6): 1017-23), cyclo(1-6)CRPRLC-KH-cyclo(9-14)CRPRLC, which blocks Apelin-induced signaling and self-renewal in GSCs.

In another preferred embodiment, the tested compound is an anti-sense nucleic acid such as a siRNA or a shRNA, said compounds having the following sequences:

siRNA specific for APJ: CCAUCAUGCUGACCUGUUACUUCUU, GACAACCAGUCUGAGUGUGAGUACA (SEQ ID NO:10),
ACUAUGACUGGCCCUUUGGGACCUU (SEQ ID NO:11)
siRNA specific for Apelin: GGUGCAGCCCAGAGGGUCAAGGAAU (SEQ ID NO:12),
CCUCUCCCAUAAGGGACCCAUGCCU (SEQ ID NO:13),
CCUGAUGCCGCUUCCCGAUGGGAAU (SEQ ID NO:14)
shRNA specific for Apelin: TAACATTCTGTGATTCTTGGC (SEQ ID NO:15),
TTACAAACATTGAACACAGGG (SEQ ID NO:16),
AACAGGGCCTTAATATCTTTG (SEQ ID NO:17),
AAGCAGACCAATCTATGGAGG (SEQ ID NO:18),
TTTCTCTGCATTCTTCCCTGG (SEQ ID NO:19)
siRNA specific for GP130: CUCACUUGCAACAUUCUUA (SEQ ID NO:20)

In another preferred embodiment, the tested compound is an antibody that is known to inhibit the interaction between APJ and Apelin.

It is to be noted that the role of the APJ/Apelin signaling pathway in tumor angiogenesis is still matter of debate. Some studies unveiled that Apelin induces tumor angiogenesis (Rayalam S. et al, 2011), whereas others demonstrated that it induces vascular normalization that eventually induce an anti-tumor effect (Kidoya et al., 2012). Moreover, no study ever disclosed the role of APJ/Apelin pathway on the survival of CSCs, as the present invention does. Altogether, this means that the skilled person was not encouraged using inhibitors of the Apelin pathway for impairing CSCs properties.

This compound is hereafter referred to as the "compound of the invention".

Importantly, the compound of the invention will selectively target cancer stem cells (and not normal stem cells), because only these cells have developed an addiction to the APJ/GP130 pathway and to the downstream activation of the Akt/mTOR and STAT3 pathways. Moreover, blocking Apelin/APJ/GP130 signaling will not target differentiated cells (either normal or cancerous cells) because APJ is lost upon their differentiation. Consequently, the compound of the invention will selectively target CSCs while sparing healthy stem cells and differentiated cells so that they will have few side effects.

Hence, the compound of the invention could be used for treating cancer patients, preferably those suffering from breast cancer, head and neck cancer, lung cancer, ovarian cancer, pancreatic cancer, colorectal carcinoma, cervical cancer, sarcomas, brain tumors, renal cancer, prostate cancer, melanoma and skin cancers, gastric cancer, multiple myeloma, leukemia or lymphoma, that are known to involve CSCs. In a preferred embodiment, this compound is used for treating glioblastoma.

Because it may not affect non-stem cancer cells, this compound could advantageously be used in combination with at least one traditional chemotherapeutic drug, such as a DNA-damaging agent, an anti-mitotic agent, and/or an antimetabolite agent.

The said DNA-damaging agent can be an alkylating agent, a topoisomerase inhibitor and/or a DNA intercalator. The said alkylating agent is preferably selected in the group consisting of: chlorambucil, cyclophosphamide, ifosfamide, mechlorethamine, melphalan, uracil mustard, thiotepa, busulfan, carmustine, lomustine, streptozocin, carboplatin, cisplatin, satraplatin, oxaliplatin, altretamine, ET-743, XLI 19 (becatecarin), dacarbazine, chlormethine, bendamustine, trofosfamide, uramustine, fotemustine, nimustine, prednimustine, ranimustine, semustine, nedaplatin, triplatin tetranitrate, mannosulfan, treosulfan, temozolomide, carboquone, triaziquone, triethylenemelamine, and procarbazin. The said topoisomerase inhibitor is preferably selected in the group consisting of: doxorubicin (doxil), daunorubicin, epirubicin, idarubicin, anthracenedione (novantrone), mitoxantrone, mitomycin C, bleomycin, dactinomycin, plicatomycin, irinotecan (camptosar), camptothecin, rubitecan, belotecan, etoposide, teniposide, and topotecan (hycamptin). The said DNA intercalator is preferably selected in the group consisting of proflavine, doxorubicin (adriamycin), daunorubicin, dactinomycin, and thalidomide.

The said antimitotic agent is preferably selected in the group consisting of: paclitaxel (abraxane)/taxol, docetaxel (taxotere), BMS-275183, xyotax, tocosal, vinorlebine, vincristine, vinblastine, vindesine, vinzolidine, etoposide (VP-16), teniposide (VM-26), ixabepilone, larotaxel, ortataxel, tesetaxel, and ispinesib.

The said antimetabolite agent is preferably selected in the group consisting of: fluorouracil (5-FU), floxuridine (5-FUdR), methotrexate, xeloda, arranon, leucovorin, hydroxyurea, thioguanine (6-TG), mercaptopurine (6-MP), cytarabine, pentostatin, fludarabine phosphate, cladribine (2-CDA), asparaginase, gemcitabine, pemetrexed, bortezomib, aminopterin, raltitrexed, clofarabine, enocitabine, sapacitabine, and azacitidine.

Further anticancer chemotherapy, and examples of suitable therapeutic protocols, maybe found in books such as Cancer Chemotherapy and Biotherapy: Principles and Practice, 3rd ed (2001), Chabner and Longo, eds., and Handbook of Cancer Chemotherapy, 6th ed. (2003), Skeet, ed., both from Lippincott Williams & Wilkins, Philadelphia, Pa., U.S.A.; Moreover, regimens for anticancer therapies, especially chemotherapies, may be found on Web sites such as those maintained by the National Cancer Institute (www.cancer.gov), the American Society for Clinical Oncology (www.asco.org), and the National Comprehensive Cancer Network (www.nccn.org). An association of the compounds of the invention with for example anti-angiogenic molecules (such as bevazicumab) is preferably considered.

In another aspect, the present invention relates to a method for treating cancer by inhibiting Cancer Stem Cells (CSCs) properties, comprising the step of administering in a subject in need thereof an efficient amount of the compound of the invention. This compound is advantageously administered before, simultaneously or after one or more traditional chemotherapeutic drug (e.g., a DNA-damaging agent, an anti-mitotic agent, and/or an antimetabolite agent, as disclosed above).

As used herein, the term "subject" refers to any animal (e.g., a mammal), including (but not limited to) human, non-human primates, rodents, and the like, which is to be the recipient of a particular treatment. Typically, the terms "subject" and "patient" are used interchangeably in reference to a human subject.

In yet another aspect, the present invention relates to a pharmaceutical composition containing the compound of the invention and a pharmaceutically acceptable carrier. This pharmaceutical composition is used preferably for treating cancers in subjects in need thereof.

The term "pharmaceutically acceptable carriers" refer to molecular entities and compositions that do not produce any adverse, allergic, or other untoward reaction when administered to an animal, or a human, as appropriate. Veterinary uses are equally included within the invention and "pharmaceutically acceptable" formulations include formulations for both clinical and/or veterinary use. As used herein, "pharmaceutically acceptable carrier" includes any and all solvents, dispersion media, coatings, antibacterial, and antifungal agents, isotonic and absorption delaying agents, and the like. The use of such media and agents for pharmaceutically active substances is well known in the art.

As used herein with respect to these methods, the term "administering" refers to various means of introducing a composition into a subject or a patient. It is intended to include (but is not limited to) subcutaneous injection, intravenous injection, intraocular injection, intradermal injection, intramuscular injection, intraperitoneal injection, intratracheal administration, epidural administration, inhalation, intranasal administration, oral administration, sublingual administration, buccal administration, rectal administration, vaginal administration, and topical administration.

As used herein, the term "effective amount" refers to an amount that will result in the desired result and may readily be determined by one of ordinary skill in the art. The compositions of the present invention may be formulated for various means of administration. These means are well known in the art and may include, for example, injection; tablets, pills, capsules, or other solids for oral administration; nasal solutions or sprays; aerosols, inhalants; topical formulations; liposomal forms; and the like.

## EXAMPLES

### I. MATERIALS AND METHODS

#### Cell culture, conditioned media preparation and Apelin secretion

Patient-derived GSCs #1 to 4 were described previously in (Patru et al, 2010), GSCs # 5 to 17 were obtained from Hospital Laennec, Nantes, France. Briefly, tumors were dissociated using the gentleMACs Dissociator (Miltenyi), according to the manufacturer's instructions. GSCs #1 to 17 were maintained in DMEM/F12 plus N2, G5 and B27 (Life Technologies). Immortalized human brain microvascular endothelial cells (hCMEC/D3) were cultured as described previously (Weksler et al, 2005) and HEK-293T in DMEM 10% FBS plus antibiotics (Invitrogen). Conditioned media from hCMEC/D3 (EC-CM) and HEK-293T (293T-CM) were obtained from 72h-old monolayers in serum-free EBM2 (Lonza). The concentration of Apelin in CM was quantified using the human Apelin EIA Kit development kit (Phoenix Pharmaceutical) as per the manufacturer's directions.

#### Plasmids, Reagents, and Transfections

HA-APJ construct was kindly supplied by Dr. L. Zheng (Chun et al., 2008) and pORF-GP130 was from InvivoGen. Transfections were performed in HEK-293T using Turbofect (Thermo Scientific). Recombinant Apelin was from Sigma-Aldrich. The APJ antagonist, MM54, cyclo(1-6)CRPRLC-KH-cyclo(9-14)CRPRLC, was kindly supplied by Dr. Robert Glenn (Macaluso NJ et al., 2011). Stealth non-silencing control and selected siRNA for human Apelin, APJ and GP130 were from Invitrogen and Sigma: siRNA specific for APJ (CCAUCAUGCUGACCUGUUACUUCUU, GACAACCAGUCUGAGUGUGAGUACA, ACUAUGACUGGCCCUUUGGGACCUU); siRNA specific for Apelin (GGUGCAGCCCAGAGGGUCAAGGAAU, CCUCUCCCAUAAGGGACCCAUGCCU, CCUGAUGCCGCUUCCCGAUGGGAAU); siRNA specific for GP130 (CUCACUUGCAACAUUCUUA). Transfections were performed using RNAiMAX lipofectamine (Invitrogen). GIPZ Lentiviral shRNAs Apelin were purchased from Thermo Scientific (TAACATTCTGTGATTCTTGGC, TTACAAA-CATTGAACACAGGG, AACAGGGCCTTAATATCTTTG, AAGCAGACCAATCTATGGAGG, TTTCTCTGCATTCTTC-CCTGG). Lentivirus production was performed as previously described (Galan et al 2011b). 48h post-infection, puromycin selection was initiated (Sigma, 1.5 mg/ml).

**Secondary neurosphere formation**

GSCs were dissociated by up-and-down pipetting and cultured in a 48-well plate format. Cells were dissociated again at days 1 and 2 after mitogen-deprivation and then maintained until day 3. NS counts were blindly performed on five random fields of view (fov) and the mean of neurosphere/fov was calculated from 3 independent experiments. Statistical analyses were performed using 2-way Student's test (Excel Microsoft Office).

**RNA Extraction and RT-PCR**

RNA was extracted using the Qiagen RNeasy Mini Kit as per the manufacturer's directions. Equal amounts of RNA were reverse transcribed using the Superscript III RT kit (Invitrogen) and the resulting cDNA was used to amplify APJ, Gp130, Sox2, Nestin and Oct4 mRNA by PCR using gene specific primer sets (table 1) in the presence of Red Taq DNA polymerase (Sigma). Human beta-actin was also amplified as a control for input. PCR products were separated by electrophoresis on SYBR green-containing agarose gels (Invitrogen) and DNA was visualized by UV illumination (Appligene).

**Western blot and Antibodies**

Proteins were collected in TNT buffer (10 mM Tris-HCl pH 7.5, 150 mM NaCl, 1% Triton X100, 2mM EDTA) plus protease inhibitors (Sigma), 200 mM NaF and 0.1 mM Na3VO4. Equal amounts of proteins (microBCA kit, Pierce) were separated with 4-12% Nupage gels (Invitrogen) and transferred onto PVDF membranes (Thermo Fisher Scientific). Alexa680-conjugated secondary antibodies (Invitrogen) were used and membranes were scanned using the Odyssey infrared imaging system (Licor). The following antibodies were used: phospho(p)-S-Akt, pT-Akt, p-S6, Akt total, p-Y-STAT3, p-S-STAT3, STAT3 (Cell Signaling), APC-APJ, control Ig and non-conjugated APJ (R&D systems), GP130 (Abcam), Tubulin (Santa Cruz), and Sox2 and Nestin (Millipore).

**Luciferase assay**

HEK-293T cells were co-transfected with HA-APJ, GP130, STAT3 Luciferase Reporter Vector (Affymetrix) and Renilla (Promega). 24h after transfection, cells were pre-treated with the indicated drugs and 1h after exposed to Apelin during 6h. Then, cells were lysed and STAT3 luciferase activity was measured using the Dual-Luciferase Reporter Assay (Promega).

**Flow cytometry**

For cell surface expression analysis, cells were incubated with the conjugated antibody for 1h and then washed twice with cold PBS. Flow cytometry analyses were performed on Accuri cytometer (BD Biosciences, CYBIO facility, Institut Cochin) and processed using CFlow plus software (BD Biosciences).

**BRET analysis**

HEK-293T cells expressing APJ, GP130 or co-expressing both receptors together with the RLuc-βarrestin2-YFP (kindly provided by Dr. M. Scott) were distributed in poly-lysine coated 96-well white microplates 24h after transfection. Cells were then washed twice with PBS and incubated with coelenterazine h (final concentration of 50 nM in Hank's solution) for 10 min. Cells were subsequently stimulated with Apelin (1 $\mu$M) for the indicated times and light emission was detected (460-500 nm for Luc and 510-550 nm for YFP) using a multilabel reader (Mithras LB 940; Berthold Technologies). The BRET signal measuring beta-arrestin conformational change due to its recruitment was determined as the ratio of the light emitted by YFP to that by Luc. The values were corrected by subtracting the background BRET RLuc signals detected when RLuc-β arrestin-YFP was expressed alone.

**Radioactivity binding assays using membrane preparations**

For the membrane fractions preparation, HEK-293T and GSC cells were lysed (5 mM Tris pH7.4 and 2 mM EDTA) and homogenized 4 x 5 sec using ultrathorax (IKA-Labortechnik, Germany). Homogenates were cleared by 5 min centrifugation at 3000 rpm. Then cells were resuspended in binding buffer (50 nM Hepes, 5 mM MgCl2 and 1% BSA) and ultracentrifuged to obtain cell membranes. Membranes were then incubated with [125I][<Glu65,Nle75,Tyr77]apelin-13 (Perkin Elmer) in 100 μl of the binding buffer at room temperature for 90 min. In order to determine the amounts of nonspecific binding, unlabeled [<Glu65,Nle75,Tyr77] apelin-13 (Sigma) was simultaneously added After incubation, bound and free radioactivities were separated through rapid filtration using the glass-fiber filter units (GF/C, Packard Instrument Co.) of a cell harvester (Packard). Filter units were completely dried, and Microcinti O (Packard) was added to each well. The radioactivity of each well was counted with a TopCount liquid scintillation counter (Packard). The dissociation constant (Kd) and the number of binding sites (B max) were determined by the method of Scatchard.

**Receptor Binding Assays Using Intact Cells.**

Prior to binding experiments, $2x10^6$ GSCs were washed three times with DMEM/F12, and resuspended in binding buffer (DMEM/F12, 40 mM HEPES, 1% BSA). In order to determine the amount of nonspecific binding, 1 μM unlabeled [<$Glu^{65}$,$Nle^{75}$,$Tyr^{77}$]apelin-13 was added. Cells were pre-incubated with GP130 blocking antibody (2 μg/ml) when indicated. Cells were then incubated with 400 pM[$^{125}$I][<$Glu^{65}$,$Nle^{75}$,$Tyr^{77}$]apelin-13 in agitation for 6h at 4°C, and radioactivity was measured with a gamma counter (Beckman).

***In vivo* experiments**

GSC neurospheres were dissociated, washed, and resuspended in PBS:Matrigel (Growth factor reduced, BD, France) at a concentration of $1x10^6$ cells in 100μL. Four weeks old female athymic nude mice (Janvier, France) were injected with 100μL per flank (3 mice per group, two injections per mice). Treatments started four weeks after, when tumors had reached 3mm of diameter. Mice were treated twice per week with either PBS alone or APJ antagonist MM54 (cyclo(1-6)CRPRLC-KH-cyclo(9-14)CRPRLC, 20μM, 100μL final volume in PBS) during the 6 following weeks. Once per week, tumors were measured and their volumes were calculated (Volume = $(width)^2$ x length/2). At week 11, mice were sacrificed and tumors were extracted for histologic analysis.

**RESULTS**

**Endothelium-secreted Apelin sustains GSC stemness**

The 13 amino-acid peptide Apelin was found to be released, among many other proteins, in an endothelial cell conditioned media (EC-CM) (Fig. 1, 2A-B). Interestingly, recombinant Apelin was sufficient to support self-renewal in a panel of 16 patient-derived GSCs, as evidenced by secondary neurosphere formation and Sox2/Nestin expression (Fig. 2C-D). Conversely, Apelin-depleted EC-CM failed to maintain GSC self-renewal (Fig. 2G).

**Apelin maintains neurosphere integrity through APJ signaling to mTOR**

APJ, the cognate receptor for Apelin, belongs to the G protein-coupled receptor family. APJ is related to the angiotensin II receptor and has been described as a co-receptor involved in the mediation of HIV-1 neuropathogenesis, cardiovascular and neuroendocrine functions. APJ expression was detected in patient-derived self-renewing GSCs, but not upon their differentiation, at the RNA and protein level (Fig. 3A-C). APJ was notably absent from glioblastoma cell lines (Fig. 3D). Moreover, cancer stem-like cells but not normal stem cells from different tissues express APJ (Fig. 3E-F). We have previously demonstrated the importance of the Akt/mTor pathway for GSC properties. In line with this, recombinant Apelin was able to activate this pathway in a similar extend than EC-CM (Fig. 4A). More interestingly, Apelin-depleted EC-CM failed to do so (Fig. 4B), suggesting that Apelin could be a key factor for EC-mediated Akt/mTor pathway activation. Furthermore, reduction of APJ expression through RNA interference effectively hampered mTor activation, triggered by both EC-CM and recombinant Apelin (Fig 4C-D). Similarly, APJ pharmacological inhibition was able to reduce both EC-CM and Apelin-induced Akt/mTor activation (Fig. 4E). Finally, targeting APJ through either siRNA or pharmacological antagonist dramatically decreased neurosphere number and size, when GSCs were cultured with EC-CM, along with Sox2/Nestin diminution (Fig. 4F).

**Apelin co-opts GP130 to fully activate APJ-dependent mTOR pathway**

mTOR is known to phosphorylate STAT3. It was thus decided to evaluate STAT3 signaling in response to both EC-CM and recombinant Apelin. Interestingly, both recombinant and endothelial-derived Apelin were able to activate this signaling pathway (Fig. 5A). Furthermore, interfering with APJ in GSCs or Apelin in endothelial cell was sufficient to reduce STAT3 phosphorylation (Fig. 5B).

It is well described that GP130 can function as a signal transducer for many cytokine receptors involved in stemness, such as LIFR and CNTFR. Therefore, it was investigated whether GP130 could play a role as well in Apelin/APJ signaling. Interfering with GP130 expression through siRNA showed that GP130 is strictly required for EC-CM-mediated Akt/mTor pathway activation (Fig. 5C). Moreover, a GP130 blocking antibody also efficiently blocked Akt/mTor pathway activation in response to both EC-CM and recombinant Apelin (Fig. 5D). Using a similar approach, we evaluated the impact of GP130 blockade on GSC stemness. Noticeably, interfering with GP130 dramatically decreased the number and size of neurosphere cultured with EC-CM, while it barely affected those of GSCs growing in control medium (Fig. 5E). This set of data points for a role of GP130 as a signal transducer in APJ/Apelin axis.

We then deciphered the interplay between APJ and GP130 in response to Apelin stimulation. First, we observed that GP130 was strictly required for Apelin-mediated STAT3 and APJ activation, as evidenced by luciferase-based reporter and BRET assays, respectively (Fig. 6A-B). Interestingly, endogenous APJ and GP130 co-localize in GSC neurospheres (not shown). Although GP130 was not required for Apelin binding to APJ (Fig. 6C), treatment with a blocking antibody prevented from Apelin binding (Fig. 6D). Interestingly, flow cytometry analysis revealed that GP130 modulated APJ surface availability (not shown).

Finally, the effects of Apelin signaling in tumor initiation and progression were further assessed *in vivo,* using a xenograft model where human GSCs were implanted into immunocompromised mouse flanks (Fig. 6F). While vehicle treated mice show an increase of tumor volume over time, APJ antagonist MM54 intraperitoneal injections restrain tumor growth and expansion. Although the exact mechanism by which the APJ inhibitor quells tumor formation (i.e. anti-angiogenic and/or anti-tumorigenic action) is not deciphered, these results suggested that APJ signaling is required for GSC tumor-initiating properties.

**Screening assay of the invention**

The inventors highlighted an APJ - GP130 functional interplay by three different means.

- Apelin, APJ and GP130 are required for STAT3 signaling in HEK-293T cells (Luciferase STAT3 reporter assay). This activation is blocked using anti-GP130 antibodies or a pharmacological antagonist of APJ.

- GP130 is required for the Apelin-dependent APJ (G-Protein Coupled Receptor) activation in HEK-293T cells (BRET assay).

- GP130 is required for Apelin binding to APJ but does not affect Apelin affinity for total APJ in GSCs (radioactive binding assay using GP130-siRNA and anti-GP130 antibodies).

This interplay does not occur in non-cancerous stem cells, as APJ is not expressed in human fetal neural stem cells, mouse embryonic stem cells, and human hematopoietic adult stem cells (figure 3d-e).

As mentioned previously, it would be interesting to inhibit Apelin/APJ/GP130 signaling axis to interfere with the integrity of the GSC tumor population, and, therefore, with glioblastoma progression.

The inventors set up experimental conditions for identifying Apelin/APJ/GP130 pathway inhibitor(s) in high-through-put screening. This screening advantageously requires the two following independent steps.

<u>1. A primary screen based on STAT3 pathway inhibitory activity: STAT3 reporter</u>

In white 96-well plates, HEK-293T cells which do not expressed APJ and GP130 were first transfected with APJ and GP130 cDNA, together with the firefly luciferase constructs downstream of STAT3 promoter and *Renilla* luciferase control plasmid. These cells were then treated with Apelin 24 hours later. Molecule candidates were added, and the STAT3-dependent luciferase signal was measured.

Importantly, STAT3 pathway activation was specific of Apelin/APJ/GP130 pathway and integrated upstream mTor signal. Consequently, in this first step, the firefly luciferase signal was observed only in the presence of a functional interplay between Apelin, APJ and GP130.

2. Secondary screen based on APJ G-Protein Coupled Receptor activation: BRET reporter assay

In white 96-well plates, HEK-293T cells were transfected with mock, APJ and/or GP130 cDNA, together with the RLuc-beta-arrestin2-YFP plasmid (Charest PG et al, EMBO, 2005). These cells were then treated with Apelin 24 hours later. Molecule candidates were added, and the BRET-signal was measured. It was shown that the BRET signal was significantly increased and thus APJ was activated only in presence of a functional interplay between Apelin, APJ and GP130.

**Table 1. Peptidome and proteome analysis from hCMEC/D3 secretome**

MS/MS analysis was performed on hCMEC/D3 secretome and compared to the HEK-293T one. Shared hits were removed from the list. The MS score and number of hits are indicated for each of the 22 identified proteins.

| Family | Name | SwissProt ID | Score | Hits |
|---|---|---|---|---|
| Secreted Growth Factors | ADM | P35318 | 679 | 13 |
| | Apelin | Q9ULZ1 | 359 | 8 |
| | CTGF | P29279 | 58 | 2 |
| | Follistatin-like 1 | Q12841 | 55 | 1 |
| | Pentraxin related protein 3 | P26022 | 246 | 6 |
| | IGF-BP7 | Q16270 | 86 | 2 |
| | MIF | P14174 | 50 | 1 |

| | | | | |
|---|---|---|---|---|
| | TGFb2 | P61812 | 46 | 1 |
| | Galectin1 | P09382 | 49 | 1 |
| Extracellular Matrix | Galectin3 binding protein | Q08380 | 399 | 6 |
| | Fibronectin | P02751 | 543 | 12 |
| | Thrombospondin 1 | P07996 | 615 | 14 |
| | Fibulin3 | Q12805 | 181 | 4 |
| | HSPG2 | P98160 | 91 | 3 |
| | Laminin 5 | O15230 | 73 | 2 |
| | Edil3 | O43854 | 47 | 1 |
| Proteases | Serglycin | P10124 | 72 | 3 |
| | Serine protease 23 | O95084 | 62 | 2 |
| | Pai 1 | P05121 | 391 | 8 |
| | Cystatin C | P01034 | 118 | 2 |
| | MMP 1 | P03956 | 56 | 1 |
| | Cathepsin B | P07688 | 36 | 1 |

## Claims

1. A method for identifying compounds capable of inhibiting Cancer Stem Cells (CSCs) properties, said method comprising the steps:

   a) contacting cells expressing the co-receptors APJ and GP130 with the Apelin polypeptide and a candidate compound,
   b) detecting the activation level of an Apelin-dependent signaling pathway in the cells obtained in step a),
   c) comparing said activation level with the activation level of said cells contacted only with the Apelin polypeptide.

2. The method of claim 1, wherein a candidate compound is selected if the activation level of an Apelin-dependent signaling pathway is impaired in the presence of said compound.

3. The method of claim 1 or 2, wherein detecting step b) comprises the detection and/or measurement of the activation level of an APJ-dependent signaling pathway.

**4.** The method of claim 3, wherein activation of the APJ receptor is assessed by studying beta-arrestin2 recruitment to APJ, preferably by Bioluminescence Resonance Energy Transfert (BRET).

**5.** The method of claim 3, wherein said APJ-dependent signaling pathway is the Akt/mTOR signaling pathway.

**6.** The method of claim 1 or 2, wherein detecting step b) comprises the detection and/or measurement of the activation level of a GP130-dependent signaling pathway.

**7.** The method of claim 1 or 2, wherein said detecting step b) comprises the detection of the interaction between the co-receptors APJ and GP130.

**8.** The method of any one of claim 1 to 7, wherein the detecting step b) comprises the steps of:

b1) measuring the Apelin-dependent activation level of a GP130-dependent signaling pathway, and
b2) measuring the Apelin-dependent activation level of an APJ-dependent signaling pathway in these cells.

**9.** The method of claim 8, wherein the detecting step b) comprises the steps of:

b1) measuring the Apelin-dependent activation level of the STAT3 signaling pathway in said cells, and
b2) measuring the Apelin-dependent beta-arrestin2 recruitment to APJ in said cells.

**10.** The method of any one of claim 1 to 9, wherein said CSCs properties are potency, self-renewal and survival.

**11.** *An in vitro* method to inhibit CSC properties, said method comprising the step of contacting these cells with a compound that is able to:

a) inhibit the interaction of Apelin on its coreceptors APJ and/or GP130, or
b) inhibit the interaction of the coreceptors APJ and GP130, or
c) inhibit simultaneously the mTOR and STAT3 signaling pathways.

**12.** The method of claim 11, wherein said compound is chosen in the group consisting of: an anti-sens nucleic acid, a receptor decoy, an aptamer, an antibody and a small molecule antagonist.

**13.** A compound that is able to:

a) inhibit the interaction of Apelin on its coreceptors APJ and / or GP130, or
b) inhibit the interaction of the coreceptors APJ and GP130, or
c) inhibit simultaneously the mTOR and STAT3 signaling pathways,

for use for treating cancer by inhibiting CSCs properties and, preferably, GSCs properties.

**14.** The compound for use of claim 13, said compound being chosen in the group consisting of: an anti-sens nucleic acid, a receptor decoy, an aptamer, an antibody and a small molecule antagonist.

Figure 1

A. Secreted Growth Factors

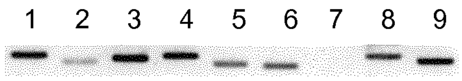


1. ADM / 2. Apelin / 3. CTGF / 4. Follistatin-like 1 / 5. Pentraxtin related protein 3 / 6. IGF-BP7 / 7. MIF / 8. TGFb2 / 9. Galectin 1

B. Proteases

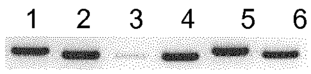


1. Cystatin C / 2. Pai 1 / 3. MMP1 / 4. Serine protease 23 / 5. Cathepsin B / 6. Serglycin

C. Extracellular matrix

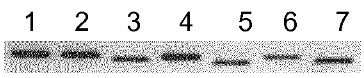


1. Galectin 3 binding protein / 2. Fibronectin / 3. Thrombospandin 1 / 4. Fibulin 3 / 5. HSPG2 / 6. Laminin 5 / 7. Edil 3

Figure 2

A

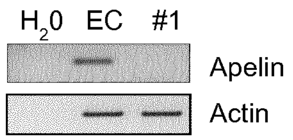
H20 EC #1
Apelin
Actin

B

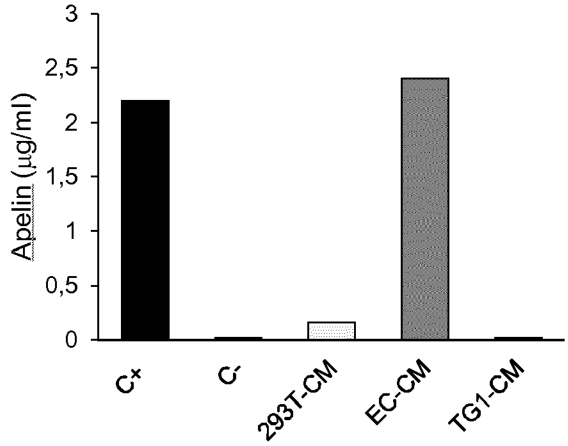
3
2,5
2
1,5
1
0,5
0
Apelin (µg/ml)
C+
C-
293T-CM
EC-CM
TG1-CM

C

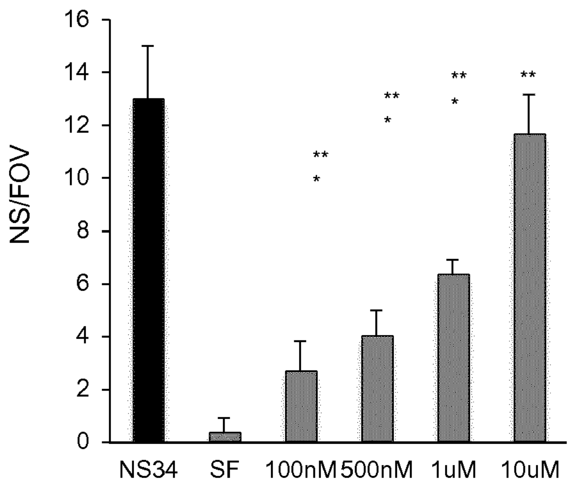
16
14
12
10
8
6
4
2
0
NS/FOV
**
*
NS34
SF
100nM
500nM
1uM
10uM

Figure 2 (continued)

D

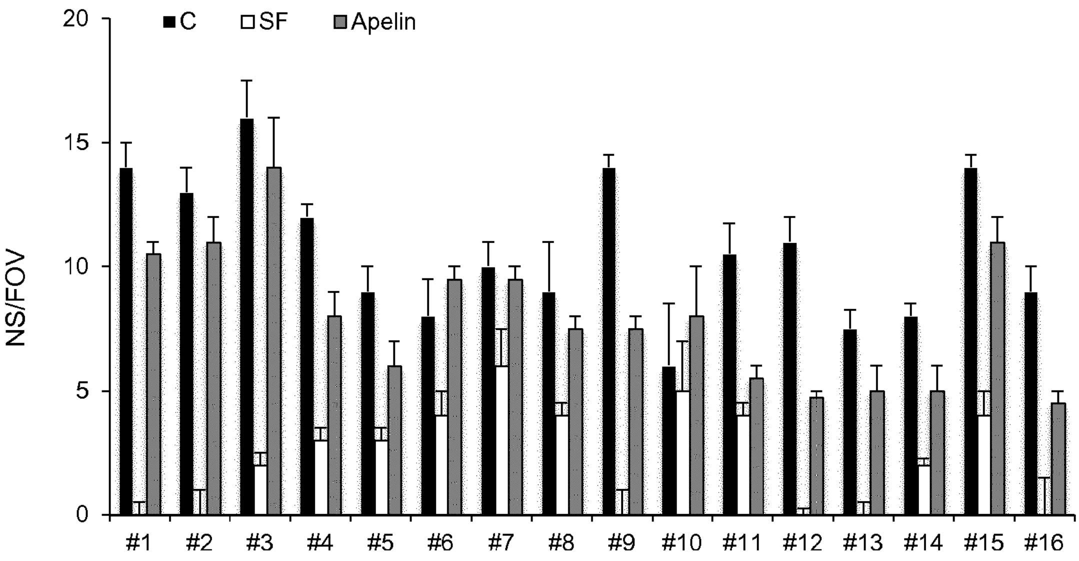


E

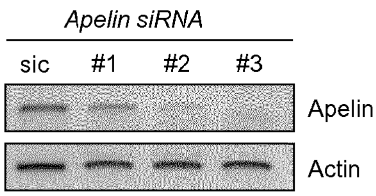


F

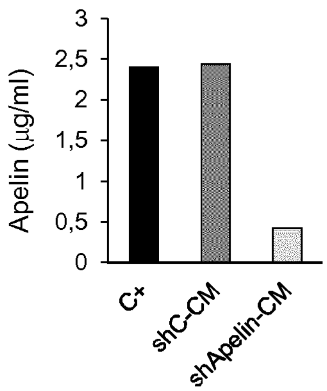


G

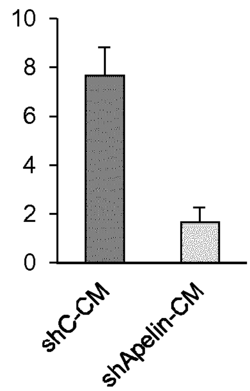

Figure 3

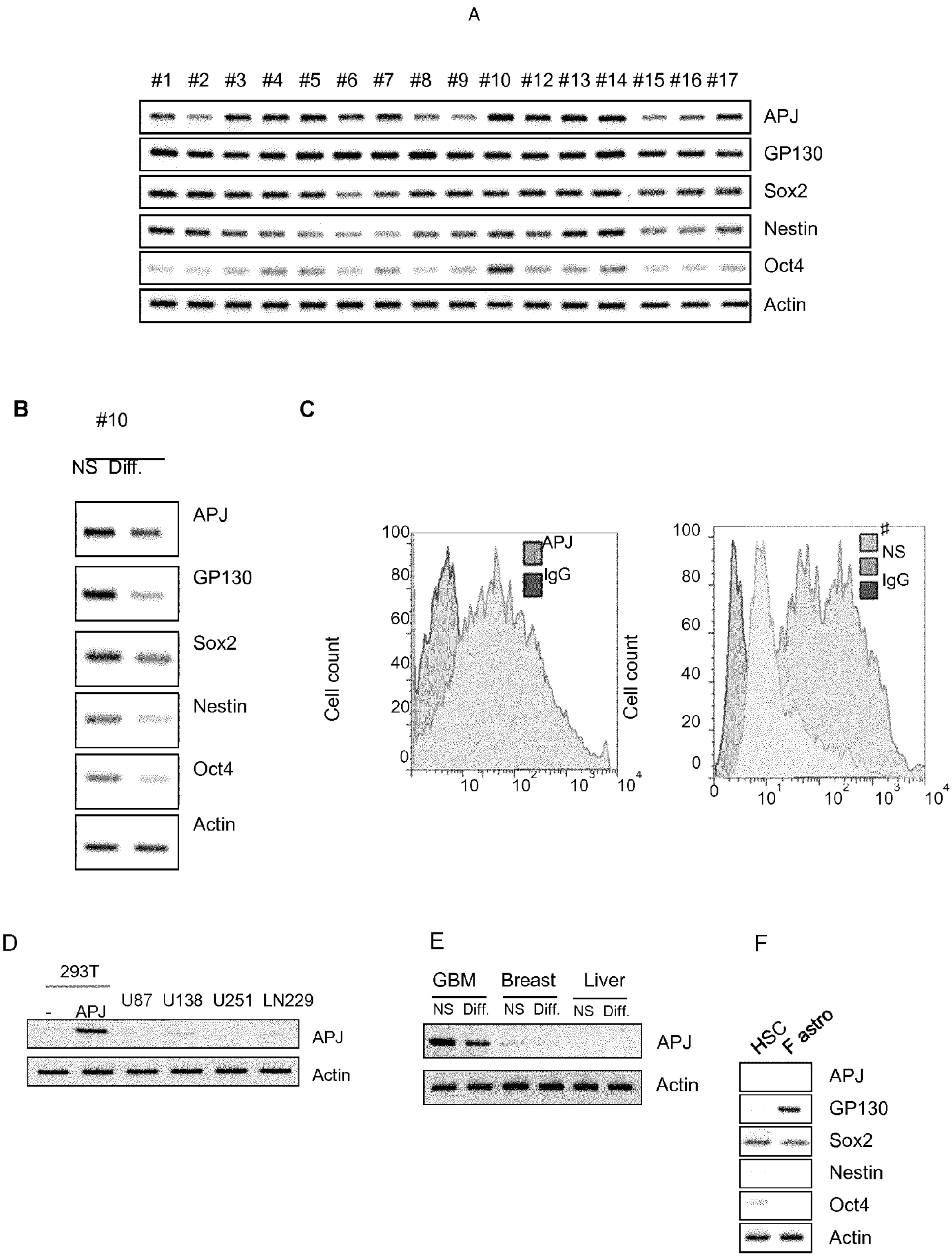
A
#1 #2 #3 #4 #5 #6 #7 #8 #9 #10 #12 #13 #14 #15 #16 #17
APJ
GP130
Sox2
Nestin
Oct4
Actin
B
#10
NS Diff.
APJ
GP130
Sox2
Nestin
Oct4
Actin
C
Cell count
APJ
IgG
NS
IgG
D
293T
- APJ
U87 U138 U251 LN229
APJ
Actin
E
GBM Breast Liver
NS Diff. NS Diff. NS Diff.
APJ
Actin
F
HSC F astro
APJ
GP130
Sox2
Nestin
Oct4
Actin

Figure 4

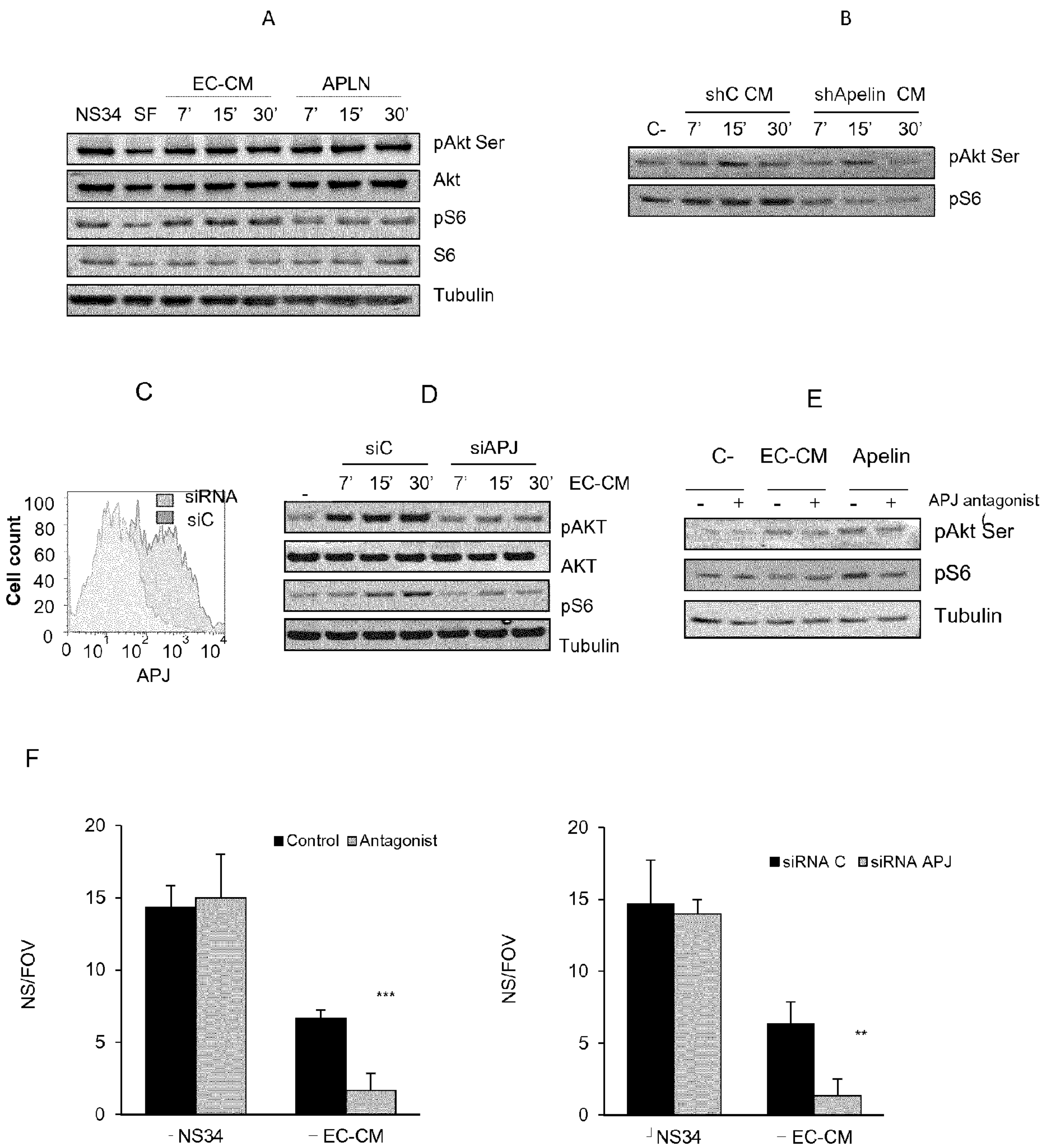
A
EC-CM
APLN
NS34 SF 7' 15' 30' 7' 15' 30'
pAkt Ser
Akt
pS6
S6
Tubulin
B
shC CM
shApelin CM
C- 7' 15' 30' 7' 15' 30'
pAkt Ser
pS6
C
siRNA
siC
Cell count
APJ
D
siC
siAPJ
- 7' 15' 30' 7' 15' 30' EC-CM
pAKT
AKT
pS6
Tubulin
E
C- EC-CM Apelin
APJ antagonist
- + - + - +
pAkt Ser
pS6
Tubulin
F
Control
Antagonist
NS/FOV
NS34
EC-CM
***
siRNA C
siRNA APJ
**

Figure 5

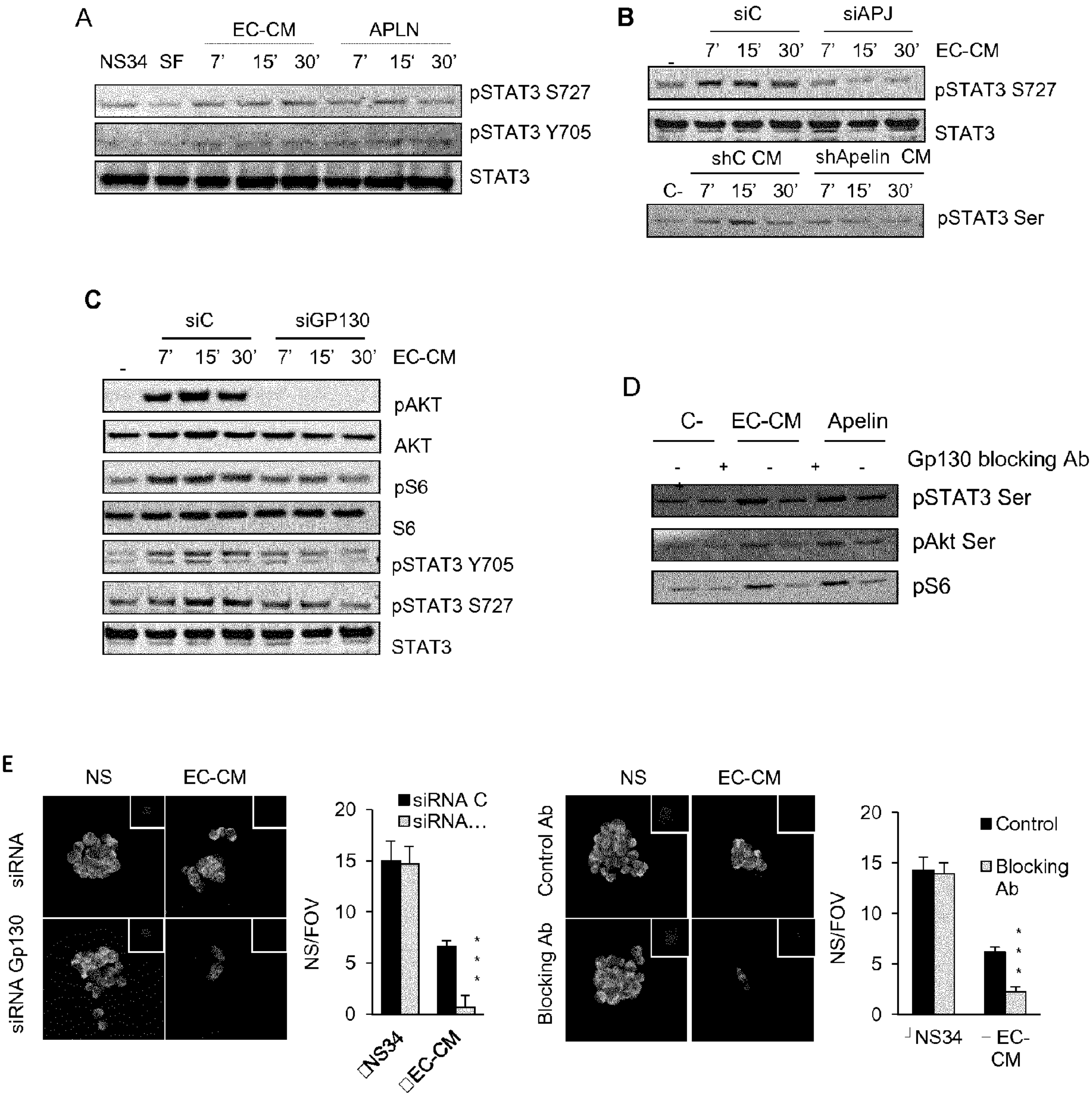
A
EC-CM
APLN
NS34 SF 7' 15' 30' 7' 15' 30'
pSTAT3 S727
pSTAT3 Y705
STAT3
B
siC
siAPJ
- 7' 15' 30' 7' 15' 30' EC-CM
pSTAT3 S727
STAT3
shC CM
shApelin CM
C- 7' 15' 30' 7' 15' 30'
pSTAT3 Ser
C
siC
siGP130
- 7' 15' 30' 7' 15' 30' EC-CM
pAKT
AKT
pS6
S6
pSTAT3 Y705
pSTAT3 S727
STAT3
D
C-
EC-CM
Apelin
- + - + -
Gp130 blocking Ab
pSTAT3 Ser
pAkt Ser
pS6
E
NS
EC-CM
siRNA
siRNA Gp130
siRNA C
siRNA...
NS/FOV
NS34
EC-CM
Control Ab
Blocking Ab
Control
Blocking Ab
NS34
EC-CM

Figure 6

A

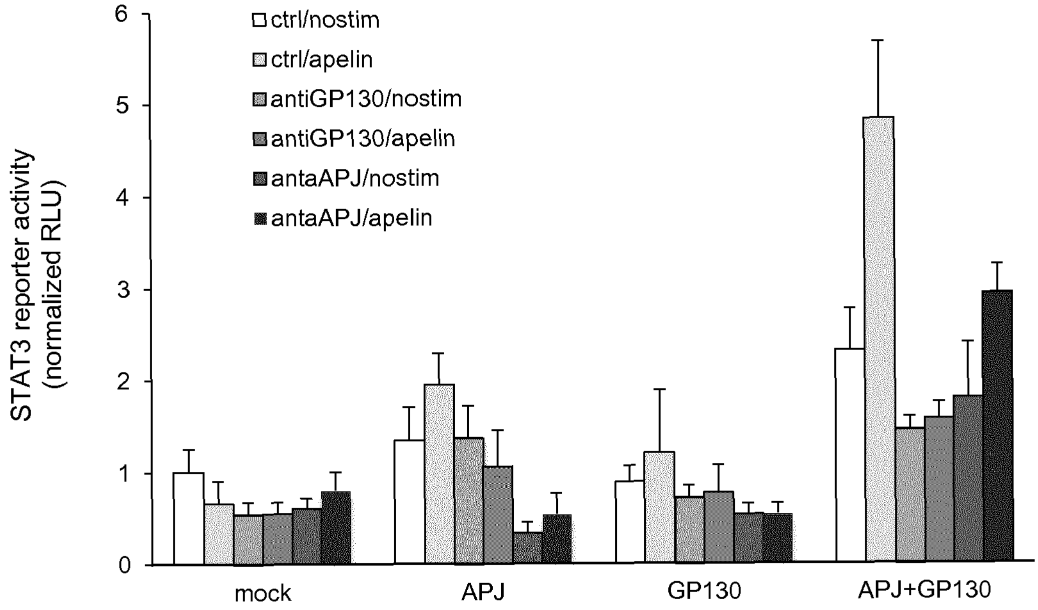
STAT3 reporter activity (normalized RLU)
6
5
4
3
2
1
0
ctrl/nostim
ctrl/apelin
antiGP130/nostim
antiGP130/apelin
antaAPJ/nostim
antaAPJ/apelin
mock
APJ
GP130
APJ+GP130

B

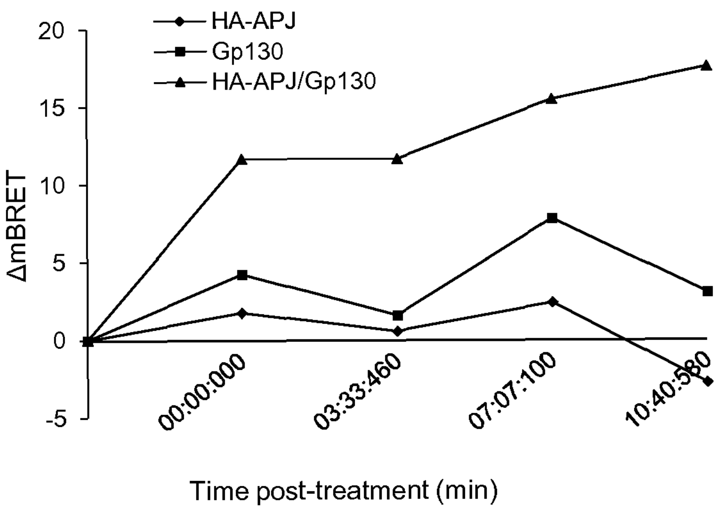
ΔmBRET
20
15
10
5
0
-5
HA-APJ
Gp130
HA-APJ/Gp130
00:00:000
03:33:460
07:07:100
10:40:580
Time post-treatment (min)

Figure 6 (continued)

C

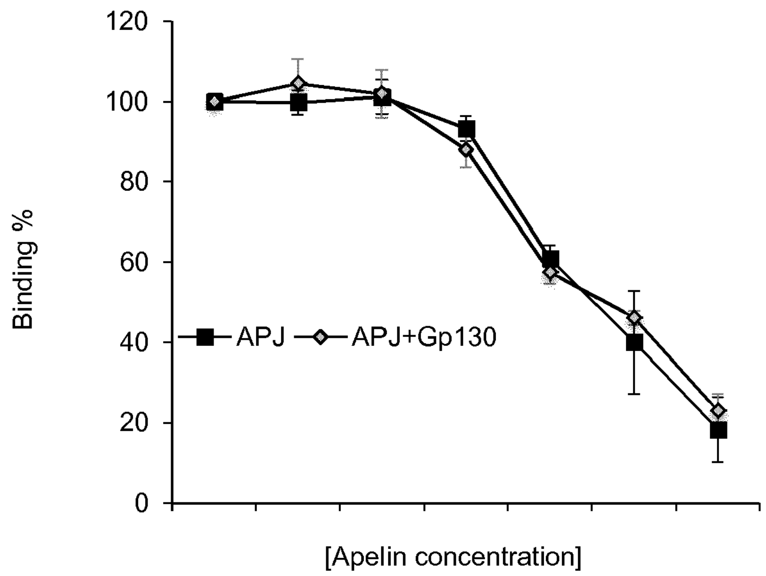


D

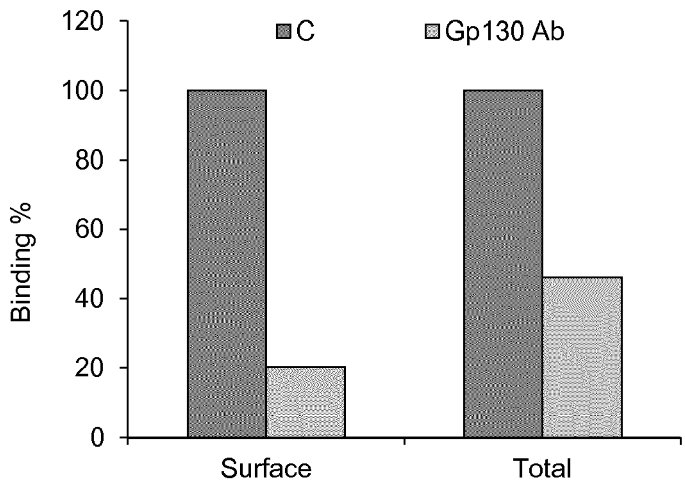

Figure 6 (continued)

F

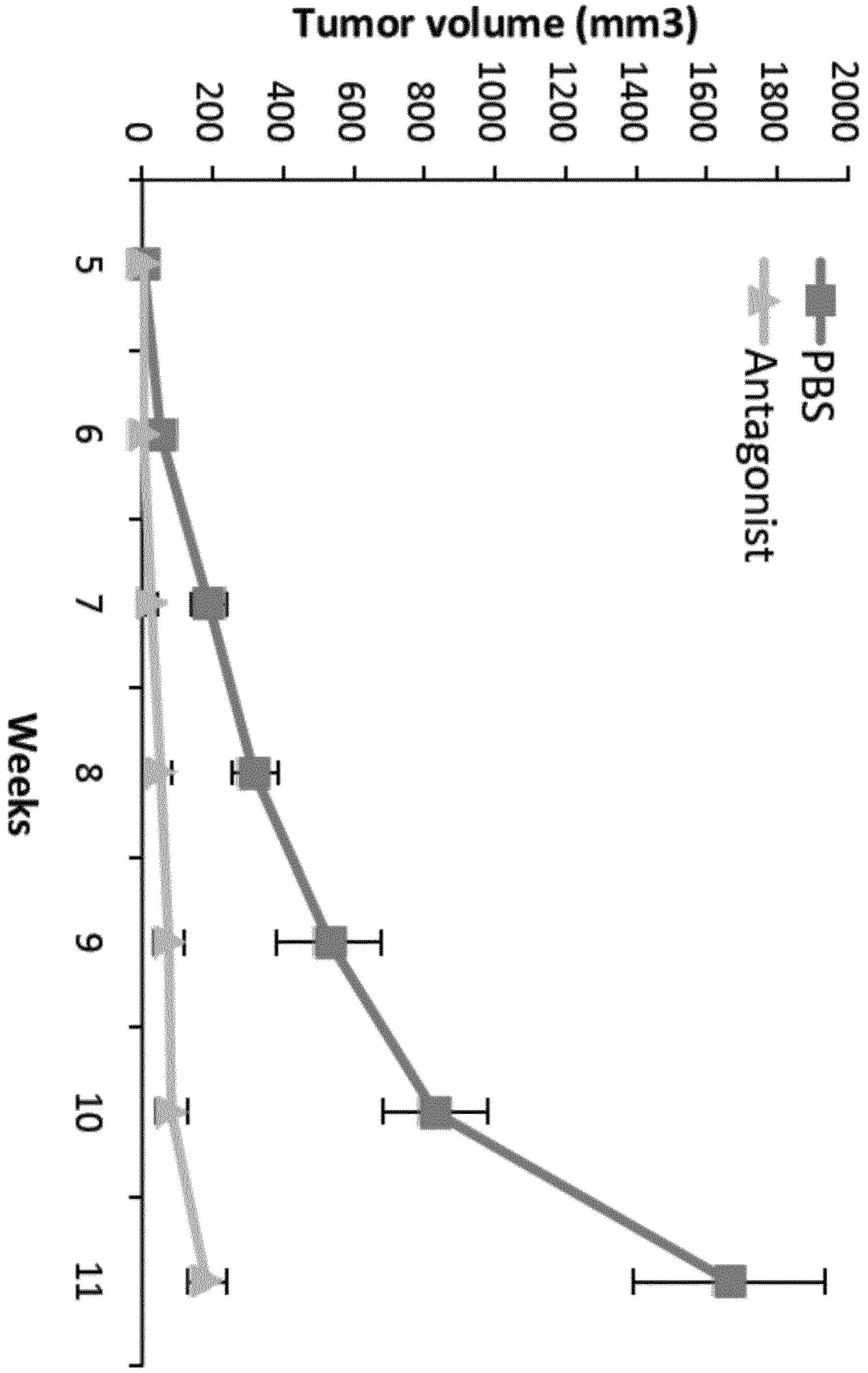

Figure 7

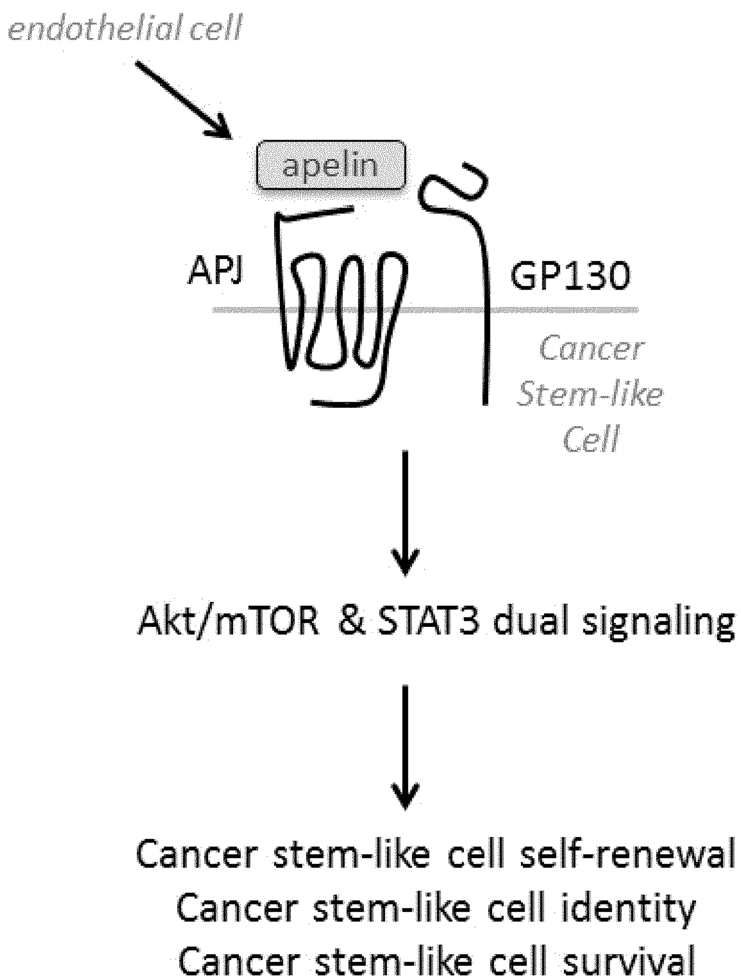
endothelial cell
apelin
APJ
GP130
Cancer
Stem-like
Cell
Akt/mTOR & STAT3 dual signaling
Cancer stem-like cell self-renewal
Cancer stem-like cell identity
Cancer stem-like cell survival

Europäisches Patentamt
European Patent Office
Office européen des brevets

# EUROPEAN SEARCH REPORT

Application Number
EP 14 30 5396

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JIANGBING ZHOU ET AL: "Activation of the PTEN/mTOR/STAT3 pathway in breast cancer stem-like cells is required for viability and maintenance", PROCEEDINGS OF THE NATIONAL ACADEMY OF SCIENCES, NATIONAL ACADEMY OF SCIENCES, vol. 104, no. 41, 9 October 2007 (2007-10-09), pages 16158-16163, XP002661526, ISSN: 0027-8424, DOI: 10.1073/PNAS.0702596104 * abstract * * page 16158, right-hand column, paragraph 2 * * page 16161 - page 16162, bridging paragraph * * page 16162, right-hand column, paragraph 3 - page 1663, left-hand column, paragraph 4 * ----- | 11-14 | INV. G01N33/50 A61P35/00 |
| A | US 2011/191868 A1 (GUPTA PIYUSH [US] ET AL) 4 August 2011 (2011-08-04) * paragraphs [0003], [0005] - [0009]; claim 1 * ----- | 1-14 | TECHNICAL FIELDS SEARCHED (IPC) G01N |
| A | HUI WANG ET AL: "Targeting Interleukin 6 Signaling Suppresses Glioma Stem Cell Survival and Tumor Growth", STEM CELLS, vol. 27, no. 10, 1 October 2009 (2009-10-01), pages 2393-2404, XP055137147, ISSN: 1066-5099, DOI: 10.1002/stem.188 * abstract * ----- | 1-14 | |

1

The present search report has been drawn up for all claims

| Place of search | Date of completion of the search | Examiner |
|---|---|---|
| The Hague | 3 September 2014 | Adida, Anne |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 14 30 5396

This annex lists the patent family members relating to the patent documents cited in the above-mentioned European search report.
The members are as contained in the European Patent Office EDP file on
The European Patent Office is in no way liable for these particulars which are merely given for the purpose of information.

03-09-2014

| Patent document cited in search report | Publication date | Patent family member(s) | Publication date |
|---|---|---|---|
| US 2011191868 A1 | 04-08-2011 | CA 2723648 A1 | 15-10-2009 |
| | | CN 102144163 A | 03-08-2011 |
| | | EP 2274617 A2 | 19-01-2011 |
| | | EP 2385370 A1 | 09-11-2011 |
| | | JP 2011522515 A | 04-08-2011 |
| | | US 2011191868 A1 | 04-08-2011 |
| | | WO 2009126310 A2 | 15-10-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **PITKIN et al.** *Pharmacological Review,* 2010
- **LEE DK. et al.** *J. Neurochem.,* 2000
- **SAMBROOK ; FITSCH ; MANIATIS.** Molecular Cloning: A Laboratory Manual. Cold Spring Harbor Laboratory Press, Cold Spring Harbor, 1989
- Current Protocols in Molecular Biology. John Wiley & Sons, Inc, 1994
- **THOMSON SP.** *Cancer Research,* 1982
- **NAKAUCHI et al.** *Ann N Y Acad Sci.,* 2001
- **AL-HAJJ et al.** *PNAS,* 2003
- **HORAN PK.** *Methods Cell Biol.,* 1990
- **DAHLROT RH. et al.** *Int. J. Clin. Exp. Pathol.,* 2013
- **ELLINGTON ; SZOSTAK.** *Nature,* 1990
- **KOHLER ; MILSTEIN.** *Nature,* 1975
- **KOHLER ; MILSTEIN.** *Eur. J. Immunol.,* 1976
- **AUSUBEL et al.** *CURRENT PROTOCOLS IN MOLECULAR BIOLOGY,* 1989
- **MACALUSO NJ et al.** *ChemMedChem,* 2011, vol. 6 (6), 1017-23
- **SCIMIA MC.** *Nature letters,* 2012
- **MACALUSO NJ et al.** *ChemMedChem,* 2011, vol. 6 (6), 1017-23
- Cancer Chemotherapy and Biotherapy: Principles and Practice. 2001
- Handbook of Cancer Chemotherapy. Lippincott Williams & Wilkins, 2003
- **CHAREST PG et al.** *EMBO,* 2005

SEQUENCE LISTING

<110>  CENTRE NATIONAL DE LA RECHERCHE SCIENTIFIQUE (CNRS)
INSTITUT NATIONAL DE LA SANTE ET DE LA RECHERCHE MEDICALE (INSERM)
UNIVERSITE PARIS DESCARTES

<120>  USE OF COMPOUNDS INHIBITING APELIN / APJ / GP130 SIGNALING

<130>  D32890 70869

<160>  20

<170>  PatentIn version 3.5

<210>  1
<211>  77
<212>  PRT
<213>  homo sapiens

<220>
<221>  MISC_FEATURE
<223>  NP_059109 - human apelin proprotein

<400>  1

```
Met Asn Leu Arg Leu Cys Val Gln Ala Leu Leu Leu Leu Trp Leu Ser
1               5                   10                  15

Leu Thr Ala Val Cys Gly Gly Ser Leu Met Pro Leu Pro Asp Gly Asn
            20                  25                  30

Gly Leu Glu Asp Gly Asn Val Arg His Leu Val Gln Pro Arg Gly Ser
        35                  40                  45

Arg Asn Gly Pro Gly Pro Trp Gln Gly Gly Arg Arg Lys Phe Arg Arg
    50                  55                  60

Gln Arg Pro Arg Leu Ser His Lys Gly Pro Met Pro Phe
65                  70                  75
```

<210>  2
<211>  13
<212>  PRT
<213>  homo sapiens

<220>
<221>  MISC_FEATURE
<223>  Apelin 13 aa  - homo sapiens

<400>  2

```
Gln Arg Pro Arg Leu Ser His Lys Gly Pro Met Pro Phe
1               5                   10
```

<210> 3
<211> 36
<212> PRT
<213> homo sapiens

<220>
<221> MISC_FEATURE
<223> Apelin 36 aa - homo sapiens

<400> 3

```
Leu Val Gln Pro Arg Gly Ser Arg Asn Gly Pro Gly Pro Trp Gln Gly
1               5                   10                  15

Gly Arg Arg Lys Phe Arg Arg Gln Arg Pro Arg Leu Ser His Lys Gly
            20                  25                  30

Pro Met Pro Phe
        35
```

<210> 4
<211> 380
<212> PRT
<213> homo sapiens

<220>
<221> MISC_FEATURE
<223> NP_005152 - human. APJ receptor

<400> 4

```
Met Glu Glu Gly Gly Asp Phe Asp Asn Tyr Tyr Gly Ala Asp Asn Gln
1               5                   10                  15

Ser Glu Cys Glu Tyr Thr Asp Trp Lys Ser Ser Gly Ala Leu Ile Pro
            20                  25                  30

Ala Ile Tyr Met Leu Val Phe Leu Leu Gly Thr Thr Gly Asn Gly Leu
        35                  40                  45

Val Leu Trp Thr Val Phe Arg Ser Ser Arg Glu Lys Arg Arg Ser Ala
    50                  55                  60

Asp Ile Phe Ile Ala Ser Leu Ala Val Ala Asp Leu Thr Phe Val Val
65                  70                  75                  80

Thr Leu Pro Leu Trp Ala Thr Tyr Thr Tyr Arg Asp Tyr Asp Trp Pro
                85                  90                  95

Phe Gly Thr Phe Phe Cys Lys Leu Ser Ser Tyr Leu Ile Phe Val Asn
            100                 105                 110
```

```
Met Tyr Ala Ser Val Phe Cys Leu Thr Gly Leu Ser Phe Asp Arg Tyr
        115                 120                 125

Leu Ala Ile Val Arg Pro Val Ala Asn Ala Arg Leu Arg Leu Arg Val
    130                 135                 140

Ser Gly Ala Val Ala Thr Ala Val Leu Trp Val Leu Ala Ala Leu Leu
145                 150                 155                 160

Ala Met Pro Val Met Val Leu Arg Thr Thr Gly Asp Leu Glu Asn Thr
                165                 170                 175

Thr Lys Val Gln Cys Tyr Met Asp Tyr Ser Met Val Ala Thr Val Ser
            180                 185                 190

Ser Glu Trp Ala Trp Glu Val Gly Leu Gly Val Ser Ser Thr Thr Val
        195                 200                 205

Gly Phe Val Val Pro Phe Thr Ile Met Leu Thr Cys Tyr Phe Phe Ile
    210                 215                 220

Ala Gln Thr Ile Ala Gly His Phe Arg Lys Glu Arg Ile Glu Gly Leu
225                 230                 235                 240

Arg Lys Arg Arg Arg Leu Leu Ser Ile Ile Val Val Leu Val Val Thr
                245                 250                 255

Phe Ala Leu Cys Trp Met Pro Tyr His Leu Val Lys Thr Leu Tyr Met
            260                 265                 270

Leu Gly Ser Leu Leu His Trp Pro Cys Asp Phe Asp Leu Phe Leu Met
        275                 280                 285

Asn Ile Phe Pro Tyr Cys Thr Cys Ile Ser Tyr Val Asn Ser Cys Leu
    290                 295                 300

Asn Pro Phe Leu Tyr Ala Phe Phe Asp Pro Arg Phe Arg Gln Ala Cys
305                 310                 315                 320

Thr Ser Met Leu Cys Cys Gly Gln Ser Arg Cys Ala Gly Thr Ser His
                325                 330                 335

Ser Ser Ser Gly Glu Lys Ser Ala Ser Tyr Ser Ser Gly His Ser Gln
            340                 345                 350

Gly Pro Gly Pro Asn Met Gly Lys Gly Gly Glu Gln Met His Glu Lys
        355                 360                 365
```

```
Ser Ile Pro Tyr Ser Gln Glu Thr Leu Val Val Asp
    370                 375                 380


<210>  5
<211>  3905
<212>  DNA
<213>  homo sapiens


<220>
<221>  misc_feature
<223>  NM_005161 - human APJ receptor mRNA

<400>  5
ggaaagccga cttgcaaaac cacagataat gttcagccca gcacagtagg ggtcaatttg        60

gtccacttgc tcagtgacaa aaagaaaaaa aaagtgggct gtcactaaag attttgactc       120

acaagagagg ggctggtctg gaggtgggag gagggagtga cgagtcaagg aggagacagg       180

gacgcaggag ggtgcaagga agtgtcttaa ctgagacggg ggtaaggcaa gagagggtgg       240

aggaaattct gcaggagaca ggcttcctcc agggtctgga gaacccagag gcagctcctc       300

ctgagtgctg ggaaggactc tgggcatctt cagcccttct tactctctga ggctcaagcc       360

agaaattcag gctgcttgca gagtgggtga cagagccacg gagctggtgt ccctgggacc       420

ctctgcccgt cttctctcca ctccccagca tggaggaagg tggtgatttt gacaactact       480

atggggcaga caaccagtct gagtgtgagt acacagactg gaaatcctcg gggggccctca      540

tccctgccat ctacatgttg gtcttcctcc tgggcaccac gggcaacggt ctggtgctct       600

ggaccgtgtt tcggagcagc cgggagaaga ggcgctcagc tgatatcttc attgctagcc       660

tggcggtggc tgacctgacc ttcgtggtga cgctgcccct gtgggctacc tacacgtacc       720

gggactatga ctggcccttt gggaccttct tctgcaagct cagcagctac ctcatcttcg       780

tcaacatgta cgccagcgtc ttctgcctca ccggcctcag cttcgaccgc tacctggcca       840

tcgtgaggcc agtggccaat gctcggctga ggctgcgggt cagcggggcc gtggccacgg       900

cagttctttg ggtgctggcc gccctcctgg ccatgcctgt catggtgtta cgcaccaccg       960

gggacttgga gaacaccact aaggtgcagt gctacatgga ctactccatg gtggccactg      1020

tgagctcaga gtgggcctgg gaggtgggcc ttggggtctc gtccaccacc gtgggctttg      1080

tggtgccctt caccatcatg ctgacctgtt acttcttcat cgcccaaacc atcgctggcc      1140

acttccgcaa ggaacgcatc gagggcctgc ggaagcggcg ccggctgctc agcatcatcg      1200

tggtgctggt ggtgaccttt gccctgtgct ggatgcccta ccacctggtg aagacgctgt      1260

acatgctggg cagcctgctg cactggccct gtgactttga cctcttcctc atgaacatct      1320

tcccctactg cacctgcatc agctacgtca acagctgcct caaccccttc ctctatgcct      1380
```

ttttcgaccc ccgcttccgc caggcctgca cctccatgct ctgctgtggc cagagcaggt 1440
gcgcaggcac ctcccacagc agcagtgggg agaagtcagc cagctactct tcggggcaca 1500
gccaggggcc cggccccaac atgggcaagg gtggagaaca gatgcacgag aaatccatcc 1560
cctacagcca ggagaccctt gtggttgact agggctggga gcagagagaa gcctggcgcc 1620
ctcggccctc cccggccttt gcccttgctt tctgaaaatc aggtagtgtg gctactcctt 1680
gtcctatgca catcctttaa ctgtcccctg attctgcccc gccctgtcct cctctactgc 1740
tttattcttt ctcagaggtt tgtggtttag gggaaagaga ctgggctcta cagacctgac 1800
cctgcacaag ccatttaatc tcactcagcc tcagtttctc cattggtatg aaatggggga 1860
aagtcatatt gatcctaaaa tgttgaagcc tgagtctgga cgcagtaaaa gcttgtttcc 1920
ctctgctgct ttcttagatc tgcaatcgtc tttcctccct tctttccttg tagtttttcc 1980
cccaccactc tctgcagctg ccgctcctta tccctgcctt ctggcaccaa tcccctccta 2040
cagctcgtcc ccctccctcc atccatcctt ctcccctgtc tactttcttg ttctgaaggg 2100
ctactaaggg ttaaggatcc caaagcttgc agagactgac cctgtttaag ctttctatcc 2160
tgttttctga gtgtgaggca gggaatgggc tggggccggg ggtgggctgt gtgtcagcag 2220
ataattagtg ctccagccct tagatctggg agctccagag cttgccctaa aattggatca 2280
cttccctgtc attttgggca ttggggctag tgtgattcct gcagttcccc catggcacca 2340
tgacactgac tagatatgct ttctccaaat tgtccgcaga ccctttcatc cttcctctat 2400
tttctatgag aattggaagg cagcagggct gatgaatgga tgtactcctt ggtttcatta 2460
tgtgagtggg gagttgggaa gggcaactag agagagagga tggaggggtg tctgcattta 2520
gtccagacac tgcttggctc gctccccgag tcctcctgtt tctgacttcc tgcataactg 2580
tgagctgaag ggtttcctca tctccccatc ttaccccatc atactgattt ctttcttggg 2640
cactggtgct acttggtgcc aagaatcatg ttgtttggga tggagatgcc tgcctcttgt 2700
ctgtgtgtgt tgtacttata tgtctatatg gatgagcctg gcatgaacag cagtgtgcct 2760
gggtcatttg gacaaatctc ctcccacccc ccaatccact gcaactctgc tgttcacaca 2820
ttacccttgg caggggggtgg tgggggcag ggacacactg aggcaatgaa aaatgtagaa 2880
taaaaatgag tccacccccct actggatttg gggctccaa cggctggtcc gtgctttagg 2940
agcgaagtta atgtttgcac caggcttcct gtagggagat ccctccccaa agcagctggc 3000
gccaaggctt gggggcgtcc tactgagctg ggttcctgct ccttcttggg ctccatgaag 3060
gaagtaagag gctagttgag agcctccctt ggccccttc cggtgcctcc ccgcctggct 3120
tcaaatttat gagcattgcc ctcatcgtcc tttcttgttc cagggtcagt ggccctcttc 3180
ctaaggaggc ctcctgcttg ccatgggcca aaaggcacgg ggtgggtttt ttctctccct 3240
accctcagga ttggacctct tggcttctgc tggattgggg atctgggaat agggactgga 3300

gcaagtgtgc agatagcatg atgtctacac tgccagagag accgtgagga tgaaattaat 3360

agtggggcct ttgtgagcta gaggctggga gtgtctattc cgggttttgt tcttggagga 3420

ctatgaaagt gaaggacaag acatgagcga tggagataag aaaagcccag cttgatgtga 3480

atggacatct tgaccctccc tggaatgacg ccagctctgg gggcagaggg aggaggagag 3540

gggaaggggc tcctcacagc ctagtctccc catcttaaga tagcatcttt cacagagtca 3600

cctcctctgc ccagagctgt cctcaaagca tccagtgaac actggaagag gcttctagaa 3660

gggaagaaat tgtccctctg aggccgccgt gggtgacctg cagagacttc ctgcctggaa 3720

ctcatctgtg aactgggaca gaagcagagg aggctgcctg ctgtgatacc cccttacctc 3780

ccccagtgcc ttcttcagaa tatctgcact gtcttctgat cctgttagtc actgtggttc 3840

atcaaataaa actgtttgtg caactgttgt gtccaaaaaa aaaaaaaaaa aaaaaaaaaa 3900

aaaaa 3905

<210> 6
<211> 918
<212> PRT
<213> homo sapiens

<220>
<221> MISC_FEATURE
<223> P40189.2 - human GP130 protein

<400> 6

Met Leu Thr Leu Gln Thr Trp Leu Val Gln Ala Leu Phe Ile Phe Leu
1 5 10 15

Thr Thr Glu Ser Thr Gly Glu Leu Leu Asp Pro Cys Gly Tyr Ile Ser
20 25 30

Pro Glu Ser Pro Val Val Gln Leu His Ser Asn Phe Thr Ala Val Cys
35 40 45

Val Leu Lys Glu Lys Cys Met Asp Tyr Phe His Val Asn Ala Asn Tyr
50 55 60

Ile Val Trp Lys Thr Asn His Phe Thr Ile Pro Lys Glu Gln Tyr Thr
65 70 75 80

Ile Ile Asn Arg Thr Ala Ser Ser Val Thr Phe Thr Asp Ile Ala Ser
85 90 95

Leu Asn Ile Gln Leu Thr Cys Asn Ile Leu Thr Phe Gly Gln Leu Glu
100 105 110

```
Gln Asn Val Tyr Gly Ile Thr Ile Ile Ser Gly Leu Pro Pro Glu Lys
        115                 120                 125

Pro Lys Asn Leu Ser Cys Ile Val Asn Glu Gly Lys Lys Met Arg Cys
    130                 135                 140

Glu Trp Asp Gly Gly Arg Glu Thr His Leu Glu Thr Asn Phe Thr Leu
145                 150                 155                 160

Lys Ser Glu Trp Ala Thr His Lys Phe Ala Asp Cys Lys Ala Lys Arg
                165                 170                 175

Asp Thr Pro Thr Ser Cys Thr Val Asp Tyr Ser Thr Val Tyr Phe Val
            180                 185                 190

Asn Ile Glu Val Trp Val Glu Ala Glu Asn Ala Leu Gly Lys Val Thr
        195                 200                 205

Ser Asp His Ile Asn Phe Asp Pro Val Tyr Lys Val Lys Pro Asn Pro
    210                 215                 220

Pro His Asn Leu Ser Val Ile Asn Ser Glu Glu Leu Ser Ser Ile Leu
225                 230                 235                 240

Lys Leu Thr Trp Thr Asn Pro Ser Ile Lys Ser Val Ile Ile Leu Lys
                245                 250                 255

Tyr Asn Ile Gln Tyr Arg Thr Lys Asp Ala Ser Thr Trp Ser Gln Ile
            260                 265                 270

Pro Pro Glu Asp Thr Ala Ser Thr Arg Ser Ser Phe Thr Val Gln Asp
        275                 280                 285

Leu Lys Pro Phe Thr Glu Tyr Val Phe Arg Ile Arg Cys Met Lys Glu
    290                 295                 300

Asp Gly Lys Gly Tyr Trp Ser Asp Trp Ser Glu Glu Ala Ser Gly Ile
305                 310                 315                 320

Thr Tyr Glu Asp Arg Pro Ser Lys Ala Pro Ser Phe Trp Tyr Lys Ile
                325                 330                 335

Asp Pro Ser His Thr Gln Gly Tyr Arg Thr Val Gln Leu Val Trp Lys
            340                 345                 350

Thr Leu Pro Pro Phe Glu Ala Asn Gly Lys Ile Leu Asp Tyr Glu Val
        355                 360                 365
```

```
Thr Leu Thr Arg Trp Lys Ser His Leu Gln Asn Tyr Thr Val Asn Ala
    370                 375                 380

Thr Lys Leu Thr Val Asn Leu Thr Asn Asp Arg Tyr Leu Ala Thr Leu
385                 390                 395                 400

Thr Val Arg Asn Leu Val Gly Lys Ser Asp Ala Ala Val Leu Thr Ile
                405                 410                 415

Pro Ala Cys Asp Phe Gln Ala Thr His Pro Val Met Asp Leu Lys Ala
            420                 425                 430

Phe Pro Lys Asp Asn Met Leu Trp Val Glu Trp Thr Thr Pro Arg Glu
        435                 440                 445

Ser Val Lys Lys Tyr Ile Leu Glu Trp Cys Val Leu Ser Asp Lys Ala
    450                 455                 460

Pro Cys Ile Thr Asp Trp Gln Gln Glu Asp Gly Thr Val His Arg Thr
465                 470                 475                 480

Tyr Leu Arg Gly Asn Leu Ala Glu Ser Lys Cys Tyr Leu Ile Thr Val
                485                 490                 495

Thr Pro Val Tyr Ala Asp Gly Pro Gly Ser Pro Glu Ser Ile Lys Ala
            500                 505                 510

Tyr Leu Lys Gln Ala Pro Pro Ser Lys Gly Pro Thr Val Arg Thr Lys
        515                 520                 525

Lys Val Gly Lys Asn Glu Ala Val Leu Glu Trp Asp Gln Leu Pro Val
    530                 535                 540

Asp Val Gln Asn Gly Phe Ile Arg Asn Tyr Thr Ile Phe Tyr Arg Thr
545                 550                 555                 560

Ile Ile Gly Asn Glu Thr Ala Val Asn Val Asp Ser Ser His Thr Glu
                565                 570                 575

Tyr Thr Leu Ser Ser Leu Thr Ser Asp Thr Leu Tyr Met Val Arg Met
            580                 585                 590

Ala Ala Tyr Thr Asp Glu Gly Gly Lys Asp Gly Pro Glu Phe Thr Phe
        595                 600                 605

Thr Thr Pro Lys Phe Ala Gln Gly Glu Ile Glu Ala Ile Val Val Pro
```

```
    610                 615                 620

Val Cys Leu Ala Phe Leu Leu Thr Thr Leu Leu Gly Val Leu Phe Cys
625                 630                 635                 640

Phe Asn Lys Arg Asp Leu Ile Lys Lys His Ile Trp Pro Asn Val Pro
                645                 650                 655

Asp Pro Ser Lys Ser His Ile Ala Gln Trp Ser Pro His Thr Pro Pro
            660                 665                 670

Arg His Asn Phe Asn Ser Lys Asp Gln Met Tyr Ser Asp Gly Asn Phe
        675                 680                 685

Thr Asp Val Ser Val Val Glu Ile Glu Ala Asn Asp Lys Lys Pro Phe
    690                 695                 700

Pro Glu Asp Leu Lys Ser Leu Asp Leu Phe Lys Lys Glu Lys Ile Asn
705                 710                 715                 720

Thr Glu Gly His Ser Ser Gly Ile Gly Gly Ser Ser Cys Met Ser Ser
                725                 730                 735

Ser Arg Pro Ser Ile Ser Ser Ser Asp Glu Asn Glu Ser Ser Gln Asn
            740                 745                 750

Thr Ser Ser Thr Val Gln Tyr Ser Thr Val Val His Ser Gly Tyr Arg
        755                 760                 765

His Gln Val Pro Ser Val Gln Val Phe Ser Arg Ser Glu Ser Thr Gln
    770                 775                 780

Pro Leu Leu Asp Ser Glu Glu Arg Pro Glu Asp Leu Gln Leu Val Asp
785                 790                 795                 800

His Val Asp Gly Gly Asp Gly Ile Leu Pro Arg Gln Gln Tyr Phe Lys
                805                 810                 815

Gln Asn Cys Ser Gln His Glu Ser Ser Pro Asp Ile Ser His Phe Glu
            820                 825                 830

Arg Ser Lys Gln Val Ser Ser Val Asn Glu Glu Asp Phe Val Arg Leu
        835                 840                 845

Lys Gln Gln Ile Ser Asp His Ile Ser Gln Ser Cys Gly Ser Gly Gln
    850                 855                 860
```

Met Lys Met Phe Gln Glu Val Ser Ala Ala Asp Ala Phe Gly Pro Gly
865 870 875 880

Thr Glu Gly Gln Val Glu Arg Phe Glu Thr Val Gly Met Glu Ala Ala
885 890 895

Thr Asp Glu Gly Met Pro Lys Ser Tyr Leu Pro Gln Thr Val Arg Gln
900 905 910

Gly Gly Tyr Met Pro Gln
915

<210> 7
<211> 5928
<212> DNA
<213> homo sapiens

<220>
<221> misc_feature
<223> NM_000565.3 - human GP130 mRNA

<400> 7
ggcggtcccc tgttctcccc gctcaggtgc ggcgctgtgg caggaagcca ccccctcggt 60

cggccggtgc gcggggctgt tgcgccatcc gctccggctt tcgtaaccgc accctgggac 120

ggcccagaga cgctccagcg cgagttcctc aaatgttttc ctgcgttgcc aggaccgtcc 180

gccgctctga gtcatgtgcg agtgggaagt cgcactgaca ctgagccggg ccagagggag 240

aggagccgag cgcggcgcgg ggccgaggga ctcgcagtgt gtgtagagag ccgggctcct 300

gcggatgggg gctgcccccg gggcctgagc ccgcctgccc gcccaccgcc ccgccccgcc 360

cctgccaccc ctgccgcccg gttcccatta gcctgtccgc ctctgcggga ccatggagtg 420

gtagccgagg aggaagcatg ctggccgtcg gctgcgcgct gctggctgcc ctgctggccg 480

cgccgggagc ggcgctggcc ccaaggcgct gccctgcgca ggaggtggcg agaggcgtgc 540

tgaccagtct gccaggagac agcgtgactc tgacctgccc gggggtagag ccggaagaca 600

atgccactgt tcactgggtg ctcaggaagc cggctgcagg ctcccacccc agcagatggg 660

ctggcatggg aaggaggctg ctgctgaggt cggtgcagct ccacgactct ggaaactatt 720

catgctaccg ggccggccgc ccagctggga ctgtgcactt gctggtggat gttccccccg 780

aggagcccca gctctcctgc ttccggaaga gccccctcag caatgttgtt tgtgagtggg 840

gtcctcggag caccccatcc ctgacgacaa aggctgtgct cttggtgagg aagtttcaga 900

acagtccggc cgaagacttc caggagccgt gccagtattc ccaggagtcc cagaagttct 960

cctgccagtt agcagtcccg gagggagaca gctctttcta catagtgtcc atgtgcgtcg 1020

ccagtagtgt cgggagcaag ttcagcaaaa ctcaaacctt tcagggttgt ggaatcttgc 1080

agcctgatcc gcctgccaac atcacagtca ctgccgtggc cagaaacccc cgctggctca 1140
gtgtcacctg gcaagacccc cactcctgga actcatcttt ctacagacta cggtttgagc 1200
tcagatatcg ggctgaacgg tcaaagacat tcacaacatg gatggtcaag gacctccagc 1260
atcactgtgt catccacgac gcctggagcg gcctgaggca cgtggtgcag cttcgtgccc 1320
aggaggagtt cgggcaaggc gagtggagcg agtggagccc ggaggccatg ggcacgcctt 1380
ggacagaatc caggagtcct ccagctgaga acgaggtgtc caccccccatg caggcactta 1440
ctactaataa agacgatgat aatattctct tcagagattc tgcaaatgcg acaagcctcc 1500
cagtgcaaga ttcttcttca gtaccactgc ccacattcct ggttgctgga gggagcctgg 1560
ccttcggaac gctcctctgc attgccattg ttctgaggtt caagaagacg tggaagctgc 1620
gggctctgaa ggaaggcaag acaagcatgc atccgccgta ctctttgggg cagctggtcc 1680
cggagaggcc tcgacccacc ccagtgcttg ttcctctcat ctccccaccg gtgtccccca 1740
gcagcctggg gtctgacaat acctcgagcc acaaccgacc agatgccagg gacccacgga 1800
gcccttatga catcagcaat acagactact tcttccccag atagctggct gggtggcacc 1860
agcagcctgg accctgtgga tgataaaaca caaacgggct cagcaaaaga tgcttctcac 1920
tgccatgcca gcttatctca ggggtgtgcg gcctttggct tcacggaaga gccttgcgga 1980
aggttctacg ccaggggaaa atcagcctgc tccagctgtt cagctggttg aggtttcaaa 2040
cctccctttc caaatgccca gcttaaaggg gctagagtga acttgggcca ctgtgaagag 2100
aaccatatca agactctttg gacactcaca cggacactca aaagctgggc aggttggtgg 2160
gggcctcggt gtggagaagc ggctggcagc ccacccctca acacctctgc acaagctgca 2220
ccctcaggca ggtgggatgg atttccagcc aaagcctcct ccagccgcca tgctcctggc 2280
ccactgcatc gtttcatctt ccaactcaaa ctcttaaaac ccaagtgcct tagcaaattc 2340
tgtttttcta ggcctgggga cggcttttac ttaaaccgcc aaggctgggg gaagaagctc 2400
tctcctccct ttcttcccta cagttgaaaa acagctgagg gtgagtgggt gaataataca 2460
gtatctcagg gcctggtcgt tttcaacaga attataatta gttcctcatt agcattttgc 2520
taaatgtgaa tgatgatcct aggcatttgc tgaatacaga ggcaactgca ttggctttgg 2580
gttgcaggac ctcaggtgag aagcagagga aggagaggag aggggcacag ggtctctacc 2640
atcccctgta gagtgggagc tgagtggggg atcacagcct ctgaaaacca atgttctctc 2700
ttctccacct cccacaaagg agagctagca gcagggaggg cttctgccat ttctgagatc 2760
aaaacggttt tactgcagct ttgtttgttg tcagctgaac ctgggtaact agggaagata 2820
atattaagga agacaatgtg aaaagaaaaa tgagcctggc aagaatgtgt ttaaacttgg 2880
tttttaaaaa actgctgact gttttctctt gagagggtgg aatatccaat attcgctgtg 2940
tcagcataga agtaacttac ttaggtgtgg gggaagcacc ataactttgt ttagcccaaa 3000

accaagtcaa gtgaaaaagg aggaagagaa aaaatatttt cctgccaggc atggtggccc 3060
acgcacttcg ggaggtcgag gcaggaggat cacttgagtc cagaagtttg agatcagcct 3120
gggcaatgtg ataaaacccc atctctacaa aaagcataaa aattagccaa gtgtggtaga 3180
gtgtgcctga agtcccagat acttgggggg ctgaggtggg aggatctctt gagcctggga 3240
ggtcaaggct gcagtgagcc gagattgcac cactgcactc cagcctgggt gacagagcaa 3300
gtgagaccct gtctcaaaaa aagaaaaaga aaaagaaaaa atattttccc tattagagaa 3360
gagattgtgg tttcattctg tattttgttt ttgtcttaaa aagtggaaaa atagcctgcc 3420
tcttctctac tctagggaaa aaccagcgtg tgactactcc cccaggtggt tatggagagg 3480
gtgtccggtc cctgtcccag tgccgagaag gaagcctccc acgactgccc ggcagggtcc 3540
tagaaattcc ccaccctgaa agccctgagc tttctgctat caaagaggtt ttaaaaaaat 3600
cccatttaaa aaaaatccct tacctcggtg ccttcctctt tttatttagt tccttgagtt 3660
gattcagctc tgcaagaatt gaagcaggac taaatgtcta gttgtaacac catgattaac 3720
cacttcagct gacttttctg tccgagcttt gaaaattcag tggtgttagt ggttacccag 3780
ttagctctca agttatcagg gtattccaga gtggggatat gatttaaatc agccgtgtaa 3840
ccatggaccc aatatttacc agaccacaaa acttttctaa tactctaccc tcttagaaaa 3900
accaccacca tcaccagaca ggtgcgaaag gatgaaagtg accatgtttt gtttacggtt 3960
ttccaggttt aagctgttac tgtcttcagt aagccgtgat tttcattgct gggcttgtct 4020
gtagatttta gaccctattg ctgcttgagg caactcatct taggttggca aaaaggcagg 4080
atggccgggc gcggtggctc acgcctgtaa tcctagcact ttgggaggcc aaggtgggag 4140
gattgcttga gctcaggagt ttgagaccaa cctgggtaac atagtgagac accatctcta 4200
ttatgaacaa taacagttaa gaaaaaaaaa ggcaggcagg cggttatggt ggttccctcc 4260
catcccacca cataaagttt ctgagacttg agaacagcaa aatgctgtta aagggaaata 4320
ttaagaatga gaatctgcag taagggtgat tctgtgccca cagttcttca attctttata 4380
ccgttttacc cacatgtggt gttaccaaag ccgggcagaa ccatgctagc ggaagatgtg 4440
aaatccagat agctcattat tgccaagagc taggcagctt tgatctccaa attgttattg 4500
ctttcatttt tattgtaatg gaattgcttt gttttgtttt tttgtttttg tattgaagag 4560
ggttgttttc cctttatttt tcataagcta atgtaaatga agaaaaaatg tcttctctgg 4620
gctgtaggcc tggctcagcg tacacaggta tacatcctaa gctctctatg ttctctaatc 4680
tgtggtgact gaacatgtgt ctcaatgcac ggggcatttc tacctgtgtt tctgcagcac 4740
ccccactgcc ttgagtcccc agcagtgctg ttatttgcct aacacctgta gccatctgcc 4800
acgcagccag acgtgaaacg ctgagacaga gaccatttag gttaaatacg acagcttatc 4860

ctgctgggtg gggaaagtaa aaaatatgct ggttcaaggc ctaaagtaaa atgatcaata 4920

atgtttgtag cattaatgaa atattttcaa gaaatgtgtc caggggtagc actggctatg 4980

ttgacgaggc ctttggtaac tcagagagct cttggccctg atggggactt gcccttacgc 5040

tttctttatc aggctctgag ttcacacgga gcctctggca cttccctgct gtcttgggag 5100

aaaggaaact ggttgccgcg gcaggttgtg gaatctgttg ctggaaccag gctggaagcc 5160

cacctggtag tgaacagggc ccagtggggc aggctgggca tgttgtggtc tatgggtttg 5220

tttcctggag aatgttcagg aatgtcttcc cagctgcttt ggtgctgagc tctattatct 5280

cacagcacgt ccagaaggct aacccaggtg gggaggatgc tgacaccagc tccaggtgga 5340

gttggtggtc ttaatttgga gatgcagggg caacctgtga ccctttgagg caagagccct 5400

gcacccagct gtcccgtgca gccgtgggca ggggctgcac acggaggggc aggcgggcca 5460

gttcagggtc cgtgccaggc cctcctcagt gccctgtgaa ggcctcctgt cctccgtgcg 5520

gctgggcacc agcaccaggg agtttctatg gcaaccttag tgattattaa ggaacactgt 5580

cagttttatg aacatatgct caaatgaaat tctactttag gaggaaagga ttggaacagc 5640

atgtcacaag gctgttaatt aacagagaga ccttattgga tggagatcac atctgttaaa 5700

tagaatacct caactctacg ttgttttctt ggagataaat aatagtttca agtttttgtt 5760

tgtttgtttt acctaattac ctgaaagcaa ataccaaagg ctgatgtctg tatatggggc 5820

aaagggtcag tatatttttc agtgtttttt tttctaccag ctattttgca tttaaagtga 5880

acattgtgtt tggaataaat actcttaaaa aataaaaaaa aaaaaaaa 5928

<210> 8
<211> 409
<212> PRT
<213> homo sapiens

<220>
<221> MISC_FEATURE
<223> human beta-arrestin2 protein - P32121.2

<400> 8

Met Gly Glu Lys Pro Gly Thr Arg Val Phe Lys Lys Ser Ser Pro Asn
1 5 10 15

Cys Lys Leu Thr Val Tyr Leu Gly Lys Arg Asp Phe Val Asp His Leu
20 25 30

Asp Lys Val Asp Pro Val Asp Gly Val Val Leu Val Asp Pro Asp Tyr
35 40 45

Leu Lys Asp Arg Lys Val Phe Val Thr Leu Thr Cys Ala Phe Arg Tyr
50 55 60

```
Gly Arg Glu Asp Leu Asp Val Leu Gly Leu Ser Phe Arg Lys Asp Leu
65                  70                  75                  80

Phe Ile Ala Thr Tyr Gln Ala Phe Pro Pro Val Pro Asn Pro Pro Arg
                85                  90                  95

Pro Pro Thr Arg Leu Gln Asp Arg Leu Leu Arg Lys Leu Gly Gln His
            100                 105                 110

Ala His Pro Phe Phe Phe Thr Ile Pro Gln Asn Leu Pro Cys Ser Val
        115                 120                 125

Thr Leu Gln Pro Gly Pro Glu Asp Thr Gly Lys Ala Cys Gly Val Asp
    130                 135                 140

Phe Glu Ile Arg Ala Phe Cys Ala Lys Ser Leu Glu Glu Lys Ser His
145                 150                 155                 160

Lys Arg Asn Ser Val Arg Leu Val Ile Arg Lys Val Gln Phe Ala Pro
                165                 170                 175

Glu Lys Pro Gly Pro Gln Pro Ser Ala Glu Thr Thr Arg His Phe Leu
            180                 185                 190

Met Ser Asp Arg Ser Leu His Leu Glu Ala Ser Leu Asp Lys Glu Leu
        195                 200                 205

Tyr Tyr His Gly Glu Pro Leu Asn Val Asn Val His Val Thr Asn Asn
    210                 215                 220

Ser Thr Lys Thr Val Lys Lys Ile Lys Val Ser Val Arg Gln Tyr Ala
225                 230                 235                 240

Asp Ile Cys Leu Phe Ser Thr Ala Gln Tyr Lys Cys Pro Val Ala Gln
                245                 250                 255

Leu Glu Gln Asp Asp Gln Val Ser Pro Ser Ser Thr Phe Cys Lys Val
            260                 265                 270

Tyr Thr Ile Thr Pro Leu Leu Ser Asp Asn Arg Glu Lys Arg Gly Leu
        275                 280                 285

Ala Leu Asp Gly Lys Leu Lys His Glu Asp Thr Asn Leu Ala Ser Ser
    290                 295                 300

Thr Ile Val Lys Glu Gly Ala Asn Lys Glu Val Leu Gly Ile Leu Val
```

305 310 315 320

Ser Tyr Arg Val Lys Val Lys Leu Val Val Ser Arg Gly Gly Asp Val
325 330 335

Ser Val Glu Leu Pro Phe Val Leu Met His Pro Lys Pro His Asp His
340 345 350

Ile Pro Leu Pro Arg Pro Gln Ser Ala Ala Pro Glu Thr Asp Val Pro
355 360 365

Val Asp Thr Asn Leu Ile Glu Phe Asp Thr Asn Tyr Ala Thr Asp Asp
370 375 380

Asp Ile Val Phe Glu Asp Phe Ala Arg Leu Arg Leu Lys Gly Met Lys
385 390 395 400

Asp Asp Asp Tyr Asp Asp Gln Leu Cys
405

<210> 9
<211> 54
<212> DNA
<213> artificial

<220>
<223> STAT3 promoter sequence

<400> 9
tgcttcccga attcccgaat tcccgaattc ccgaattccc gaattcccga acgt 54

<210> 10
<211> 25
<212> RNA
<213> artificial

<220>
<223> siRNA specific for APJ

<400> 10
ccaucaugcu gaccuguuac uucuu 25

<210> 11
<211> 25
<212> RNA
<213> artificial

<220>
<223> siRNA specific for APJ

<400> 11
gacaaccagu cugagududa guaca 25

<210> 12
<211> 25
<212> RNA
<213> artificial

<220>
<223> siRNA specific for Apelin

<400> 12
ggugcagccc agagggucaa ggaau 25

<210> 13
<211> 25
<212> RNA
<213> artificial

<220>
<223> siRNA specific for Apelin

<400> 13
ccucucccau aagggaccca ugccu 25

<210> 14
<211> 25
<212> RNA
<213> artificial

<220>
<223> siRNA specific for Apelin

<400> 14
ccugaugccg cuucccgaug ggaau 25

<210> 15
<211> 21
<212> DNA
<213> artificial

<220>
<223> shRNA specific for Apelin

<400> 15
taacattctg tgattcttgg c 21

<210> 16
<211> 21
<212> DNA
<213> artificial

<220>
<223> shRNA specific for Apelin

<400> 16
ttacaaacat tgaacacagg g 21

<210> 17
<211> 21
<212> DNA

<213> artificial

<220>
<223> shRNA specific for Apelin

<400> 17
aacagggcct taatatcttt g 21

<210> 18
<211> 21
<212> DNA
<213> artificial

<220>
<223> shRNA specific for Apelin

<400> 18
aagcagacca atctatggag g 21

<210> 19
<211> 21
<212> DNA
<213> artificial

<220>
<223> shRNA specific for Apelin

<400> 19
tttctctgca ttcttccctg g 21

<210> 20
<211> 19
<212> RNA
<213> artificial

<220>
<223> siRNA specific for GP130

<400> 20
cucacuugca acauucuua 19